# EUROPEAN PATENT SPECIFICATION

(11) **EP 0 319 050 B1**
(45) Date of publication and mention of the grant of the patent: **12.04.1995**
(21) Application number: 88121486.0
(22) Date of filing: 21.04.1986
(51) Int. Cl.: C07H 19/16, A61K 31/70

(54) **Griseolic acid derivatives, their preparation and their use**
Griseolsäurederivate, ihre Herstellung und ihre Verwendung
Dérivés de l'acide griséolique, leur préparation et leur utilisation

(30) Priority: 19.04.1985 JP 82132/85; 27.04.1985 JP 91987/85; 27.04.1985 JP 91989/85
(43) Date of publication of application: 07.06.1989
(62) Divisional of application: 86303000.3
(73) Proprietor: SANKYO COMPANY LIMITED, Tokyo (JP)
(72) Inventor: Kaneko,Masakatsu c/o Sankyo Company Limited, Shinagawa-ku Tokyo (JP); Murofushi, Yoshinobu c/o Sankyo Company Limited, Shinagawa-ku Tokyo (JP); Kimura,Misako c/o Sankyo Company Limited, Shinagawa-ku,Tokyo (JP); Yamazaki,Mitsuo c/o Sankyo Company Limited, Shinagawa-ku,Tokyo (JP); Ijima,Yasuteru c/o Sankyo Company Limited, Shinagawa-ku,Tokyo (JP)
(74) Representative: Gibson, Christian John Robert

(56) References cited:
- EP-A- 0 143 557
- US-A- 4 167 565
- US-A- 4 460 765
- CHEMICAL ABSTRACTS, vol. 106, no. 7, 16th February 1987, page 683, abstract no.50602a, Columbus, Ohio, US; & JP-A-61 100 593 (SANKYO CO., LTD) 19-05-1986

## Description

The present invention relates to a series of novel griseolic acid derivatives, and provides processes for preparing these compounds and methods and compositions using them.

Griseolic acid is a nucleoside-type compound having an adenine base and two carboxylic acid groups. It was first disclosed in, inter alia, European Patent Specification No. 29,329A, but its structure was not, at that stage known. Its structure was first disclosed in U.S. Patent Specification No. 4,460,765. Certain derivatives of griseolic acid were subsequently disclosed in European Patent Publication No. 0143557 and this also discloses the structure of griseolic acid. Other griseolic acid derivatives, specifically dihydrodesoxygriseolic acid and its salts and esters, are disclosed in European Patent Publication No. 0162715.

In accordance with the recommendations of the International Union of Pure and Applied Chemistry (IUPAC), the compounds of the present invention are named as derivatives of griseolic acid (or of dihydrodesoxygriseolic acid), taking griseolic acid as the parent structure. The numbering system employed is shown in European Patent Publication No. 0143557.

Griseolic acid and the griseolic acid derivatives of European Patent Publications No. 0143557 and 0162715, as well as the derivatives of the present invention, have the ability to inhibit the activity of phosphodiesterases specific to various cyclic nucleotides, for example 3′,5′-cyclic adenosine monophosphate (cAMP) phosphodiesterase (PDE) or 3′,5′-cyclic guanosine monophosphate (cGMP) PDE, and can thus increase the level of the cyclic nucleotide, e.g. cAMP or cGMP, in the cells of a patient treated with such a compound.

It is well known that cAMP, which is very widely distributed in animal tissues, functions as a second messenger for and mediates the effect of a large number of hormones; as a result, cAMP has a variety of very important physiological and biochemical roles. Additionally, it is known to have an effect on or participate in: division, proliferation and differentiation of cells; the systolic system, particularly miocardia; haematopoiesis; various activities of the central nervous system; immune reactions; and the liberation of insulin and histamine. Its concentration in tissues, and hence its effect upon these various functions, depends upon the balance between the enzyme which synthesizes cAMP (i.e. adenylate cyclase) and the enzyme which decomposes cAMP, cAMP PDE. An inhibitor against cAMP PDE would increase the level of cAMP in the cells and is thus expected to have a variety of therapeutic uses, for example: in the treatment of cardiovascular problems; as an antiasthmatic agent; as a smooth muscle relaxant; as a psychotropic or neurotropic agent; as an anti-inflammatory agent; in the therapy of cancer; and as a treatment for diabetes.

The activities of other cyclic nucleotides, e.g. cGMP, have, to date, been less comprehensively investigated. However, it is believed that they have a range of activities similar to, albeit not identical with, those of cAMP. Hence, inhibition of the PDE's specific to such other cyclic nucleotides will give rise to a range of therapeutic effects similar to those arising from the inhibition of cAMP PDE. As the activities of such other cyclic nucleotides are elucidated, the need will arise for inhibitors of the PDE's associated with those other nucleotides, which inhibitors show a greater specificity to one or other of the PDE's of the other nucleotides, rather than cAMP PDE; indeed, development of such inhibitors may even assist or encourage investigation of such other cyclic nucleotides.

In addition to griseolic acid and its derivatives, other compounds known to inhibit the PDE's of cAMP and cGMP include papaverine, dipyridamole and some compounds related to the constituent bases of nucleic acids, such as theophylline or M & B 22,948 [Kukovetz et al., Naunyn-Schmiedeberg's Arch. Pharmakol., 310, 129 (1979)].

We hake now discovered a series of compounds which are related to dihydrodesoxygriseolic acid and which share the activity of griseolic acid and dihydrodesoxygriseolic acid. Certain of these compounds surprisingly have a greater activity against cGMP PDE than against cAMP PDE. Certain compounds of the invention, whilst retaining the desirable activity are of more value as intermediates in the preparation of other, related compounds.

The compounds of the present invention are compounds of formula (I): 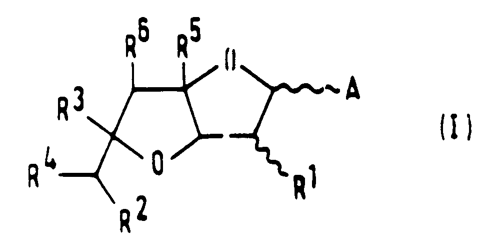
in which:
A represents a group of formula: 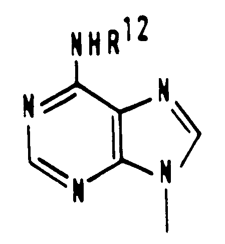
R¹ and R² are the same or different and each represents a hydrogen atom, a halogen atom or a group of formula -OR⁹;
R³ and R⁴ are the same or different and each represents a carbamoyl group or a carboxy group;
R⁵ and R⁶ both represent hydrogen atoms;
R⁹ represents a hydrogen atom, a C₁-C₆ alkyl group, an alkylsulphonyl group, a haloalkylsulphonyl group, and arylsulphonyl group or a hydroxy-protecting group;
R¹² represents a C₁-C₆ alkyl group;
and pharmaceutically acceptable salts and esters thereof.

The invention also provides a pharmaceutical composition comprising a phosphodiesterase inhibitor in admixture with a pharmaceutically acceptable carrier or diluent, wherein said phosphodiesterase inhibitor is at least one compound of formula (I), as defined above, or a pharmaceutically acceptable salt or ester thereof.

The invention further consists in the use for the manufacture of a medicament for the treatment of a disorder arising from an imbalance in phosphodiesterase levels of a compound of formula (I), as defined above, or a pharmaceutically acceptable salt or ester thereof.

The compounds of the invention may be prepared from griseolic acid or dihydrodesoxygriseolic acid by replacing the adenine moiety of said griseolic acid or dihydrodesoxygriseolic acid by another appropriate moiety of formula (A) and/or by replacing any of the groups defined above as R¹, R², R³, R⁴, R⁵, R⁶, R⁹ and R¹² by any group within the definitions of said groups.

Where reference is made herein to "aryl" groups, either as such or as part of a larger group (e.g. an arylsulphonyl, aromatic acyl, aralkyl or aralkyloxy group), this is a carbocyclic aryl group preferably having from 6 to 14, more preferably from 6 to 10, ring carbon atoms (e.g. phenyl or 1- or 2-naphthyl) which may be substituted or unsubstituted. Where the group is substituted, the substituents are preferably selected from C₁-C₄ alkyl groups, C₁-C₄ alkoxy groups, hydroxy groups, halogen atoms, nitro groups, amino groups, C₁-C₄ alkylamino groups, dialkylamino groups where each alkyl part is C₁-C₄, C₁-C₄ haloalkyl groups, C₂-C₇ alkoxycarbonyl groups, aryl groups (themselves being as defined herein, preferably phenyl groups, and substituted or unsubstituted, albeit, if substituted, preferably not with aryl groups) and cyano groups.

In the compounds of the invention, where R¹ or R² represents a halogen atom, this is suitably a fluorine, chlorine, bromine or iodine atom.

Where R¹ or R² represents a group of formula -OR⁹, R⁹ is as defined above, and hence -OR⁹ represents a hydroxy, C₁-C₆ alkoxy, alkylsulphonyloxy, haloalkylsulphonyloxy or arylsulphonyloxy group or a protected hydroxy group.

Where the group -OR⁹ represented by R¹ or R² is said C₁-C₆ alkoxy group, this may be a straight or branched chain group and examples include the methoxy, ethoxy, propoxy, isopropoxy, butoxy, isobutoxy, sec-butoxy, t-butoxy, pentyloxy and hexyloxy groups.

Where the group -OR⁹ represented by R¹ or R² represents an alkylsulphonyloxy group, this is preferably a methanesulphonyloxy, ethanesulphonyloxy or propanesulphonyloxy group.

Where the group -OR⁹ represented by R¹ or R² represents a haloalkylsulphonyloxy group, the halogen atom may be any one of those defined above in relation to R¹ and R², but is preferably a fluorine atom. One or more halogen atoms may be present, up to complete perhalogenation. The perfluoroalkylsulphonyloxy groups are particularly preferred and examples of such groups include the trifluoromethanesulphonyloxy and pentafluoroethanesulphonyloxy groups.

Where the group -OR⁹ represented by R¹ or R² represents an arylsulphonyloxy group, the aryl part is preferably as defined above and examples of such arylsulphonyloxy groups include the benzenesulphonyloxy and p-toluenesulphonyloxy groups.

Where R⁹ represents a hydroxy-protecting group, the nature of such a group is not critical to the invention, provided that it is "pharmaceutically acceptable" (i.e. it does not, or does not to an unacceptable extent, reduce the activity or increase the toxicity of the compound). Where the compound is to be used for non-therapeutic purposes (e.g. as an intermediate), however, this restriction does not apply. Examples of hydroxy-protecting groups which may be represented by R⁹ include: substituted ethyl groups; aralkyl groups; alkoxycarbonyl groups; alkenyloxycarbonyl groups; aralkyloxycarbonyl groups; heterocyclic groups having 5 or 6 ring atoms, of which from 1 to 3 are oxygen, nitrogen or sulphur hetero-atoms, said heterocyclic groups being unsubstituted or having from 1 to 3 halogen, C₁-C₄ alkyl or C₁-C₄ alkoxy substituents; trialkylsilyl groups in which each alkyl part is C₁-C₄; C₁-C₂₀ aliphatic acyl groups; aromatic acyl groups; alkoxymethyl groups; and hydroxy-protecting groups which are easily hydrolized in vivo.

Where the group -OR⁹ represented by R¹ or R² represents said substituted ethoxy group, the ethoxy group may have one or more, preferably from 1 to 3, substituents selected from C₁-C₄ alkoxy groups, C₁-C₄ alkyl groups, halogen atoms, C₁-C₄ alkylselenyl and arylselenyl groups (in which the aryl part is as defined above). Examples of such groups include the 1-ethoxyethoxy, 1-methyl-1-methoxyethoxy, 1-isopropoxyethoxy, 2,2,2-trichloroethoxy and 2-phenylselenylethoxy groups.

Where the group -OR⁹ represented by R¹ or R² represents an aralkyloxy group, the aryl part is preferably as defined above and the alkoxy part is preferably a C₁-C₄, more preferably C₁-C₃, alkoxy group. The aralkyloxy group may be a monoarylalkoxy group, a diarylalkoxy group or a triarylalkoxy group. Examples of such groups include the benzyloxy, phenethyloxy, p-methoxybenzyloxy, o-nitrobenzyloxy, p-nitrobenzyloxy, p-chlorobenzyloxy, p-cyanobenzyloxy, diphenylmethyl, triphenylmethoxy, α-naphthylmethoxy, β-naphthylmethoxy, α-naphthyldiphenylmethoxy, p-methoxyphenyl-diphenylmethoxy and 3-phenylpropoxy groups.

Where the group -OR⁹ represented by R¹ or R² represents an alkoxycarbonyloxy group, this is preferably a C₂-C₇, more preferably C₂-C₅, alkoxycarbonyloxy group (i.e. the alkoxy part is C₁-C₆, more preferably C₁-C₄) and the alkoxy part may be substituted or may have at least one substituent selected from halogen atoms and tri-substituted silyl groups (e.g. as defined below in relation to the trialkylsilyl groups). Examples of such alkoxycarbonyloxy groups include the methoxycarbonyloxy, ethoxycarbonyloxy, t-butoxycarbonyloxy, 2,2,2-trichloroethoxycarbonyloxy, isobutoxycarbonyloxy and 2-trimethylsilylethoxycarbonyloxy groups.

Where the group -OR⁹ represented by R¹ or R² represents an alkenyloxycarbonyloxy group, the alkenyl part is preferably a C₂-C₄ alkenyl group and examples include the vinyloxycarbonyloxy and allyloxycarbonyloxy groups.

Where the group -OR⁹ represented by R¹ or R² represents an aralkyloxycarbonyloxy group, the aralkyl part is preferably as described above in relation to the aralkyloxy groups which themselves may be represented by -OR⁹. Examples of such groups include the benzyloxycarbonyloxy, p-methoxybenzyloxycarbonyloxy, 3,4-dimethoxybenzyloxycarbonyloxy, o-nitrobenzyloxycarbonyloxy and p-nitrobenzyloxycarbonyloxy groups.

Where the group -OR⁹ represented by R¹ or R² represents an alkoxymethoxy group, this may be a mono-alkoxymethoxy or di-alkoxymethoxy group and the alkoxy part is preferably a C₁-C₆, more preferably C₁-C₄, alkoxy group which may be unsubstituted or have at least one substituent selected from C₁-C₄ alkoxy groups and halogen atoms. Examples of such alkoxymethoxy groups include the methoxymethoxy, ethoxymethoxy, propoxymethoxy, isopropoxymethoxy, butoxymethoxy, t-butoxymethoxy, 2-methoxyethoxymethoxy, 2,2,2-trichloroethoxymethoxy and bis(2-chloroethoxy)methoxy groups.

Where the group -OR⁹ represented by R¹ or R² represents a protected hydroxy group which is easily hydrolized in vivo, the group may fall within a number of different classes, including some classes which overlap with those protected hydroxy groups described above. In general, preferred such protected hydroxy groups include: the aralkyloxycarbonyloxy groups, particularly as defined above; and the acyloxy-substituted alkoxycarbonyloxy, preferably methoxycarbonyloxy, groups, such as the pivaloyloxymethoxycarbonyloxy group.

Where the group -OR⁹ represented by R¹ or R² represents said heterocyclic-oxy group, this is preferably such a group containing a single oxygen or sulphur hetero-atom and is more preferably a pyranyloxy, dihydropyranyloxy, tetrahydropyranyloxy or tetrahydrofuryloxy group, or their thiopyran or thiofuran analogues, which may be unsubstituted or have from 1 to 3 substituents selected from C₁-C₄ alkyl, C₁-C₄ alkoxy and halogen substituents. Preferred examples of such groups are the tetrahydropyran-2-yloxy, 3-bromotetrahydropyran-2-yloxy, tetrahydrothiopyran-2-yloxy, 4-methoxytetrahydrothiopyran-4-yloxy, tetrahydrofuran-2-yloxy, tetrahydrothie-2-yloxy and 4-methoxytetrahydropyran-4-yloxy groups.

Where the group -OR⁹ represented by R¹ or R² represents a trialkylsilyloxy group, the three alkyl groups may be the same or different and may be straight or branched chain groups containing from 1 to 4 carbon atoms. Examples of such groups are the trimethylsilyloxy, triethylsilyloxy, diisopropylmethylsilyloxy, di-t-butylmethylsilyloxy, triisopropylsilyloxy, dimethylisopropylsilyloxy and t-butyldimethylsilyloxy groups.

Where the group -OR⁹ represented by R¹ or R² represents an aliphatic acyloxy group, this is an aliphatic carboxylic acyloxy group, which may be saturated or unsaturated (the terms "saturated" and "unsaturated" referring, in this context, to carbon-carbon bonds within said groups) and there is no particular limitation on the chain length of the acyloxy group, both short and long chain acyloxy groups being useful in the present invention. Examples of such acyloxy groups include the formyloxy, acetoxy, chloroacetoxy, dichloroacetoxy, trichloroacetoxy, trifluoroacetoxy, methoxyacetoxy, propionyloxy, butyryloxy, (E)-2-methyl-2-butenoyloxy, isobutyryloxy, valeryloxy, isovaleryloxy, pivaloyloxy, octanoyloxy, lauroyloxy, palmitoyloxy and stearoyloxy groups.

Where the group -OR⁹ represented by R¹ or R² represents an aromatic acyloxy group, this is an aromatic carboxylic acyloxy group and is preferably an arylcarbonyloxy group in which the aryl part is as defined above. Preferred such aromatic acyloxy groups include the benzoyloxy, p-toluoyloxy, p-anisoyloxy, p-chlorobenzoyloxy, p-nitrobenzoyloxy, o-(dibromomethyl)benzoyloxy, o-(methoxycarbonyl)benzoyloxy, p-phenylbenzoyloxy, 2,4,6-trimethylbenzoyloxy, o-nitrobenzoyloxy and α-naphthoyloxy groups.

R¹² may be a straight or branched chain C₁₋₆ alkyl group, and examples include the methyl, ethyl, propyl, isopropyl, butyl, isobutyl, sec-butyl, t-butyl, pentyl, isopentyl, sec-pentyl, neopentyl, t-pentyl, hexyl and isohexyl groups, of which the C₁-C₄ alkyl groups are preferred.

The compounds of formula (I) contain two carboxy groups and can thus form mono- or di-salts and mono- or di-esters. In the case of the di-salts and di-esters, the cationic moieties of the salts or the alcoholic moieties of the esters can be the same or different. In practice, however, it is most easy to prepare di-salts or di-esters, particularly those in which the two cationic moieties or the two alcoholic moieties are the same.

There is no particular limitation upon the nature of the alcoholic moiety of the ester, provided that, where it is intended for therapeutic use, it is "pharmaceutically acceptable", which, as is well-known to those skilled in the art, means that is does not, or does not to an unacceptable extent, reduce the activity of the compound or increase its toxicity and all esters conventionally formed for compounds of this type may be formed with the compounds of the invention. Where the esters are intended for non-therapeutic uses (e.g. as intermediates), however, even this restriction does not apply. Examples of such esters include: C₁-C₆ alkyl esters, particularly the methyl, ethyl, propyl, isopropyl, butyl, isobutyl and t-butyl esters; aralkyl esters, particularly the benzyl, p-nitrobenzyl, o-nitrobenzyl, triphenylmethyl, bis(o-nitrophenyl)methyl, 9-anthrylmethyl, 2,4,6-trimethylbenzyl, p-bromobenzyl, p-methoxybenzyl, piperonyl and benzhydryl esters; C₁-C₆ haloalkyl esters which may have 1 or more halogen (e.g. chlorine, bromine, fluorine or iodine) atoms up to complete perhalogenation, e.g. the 2,2,2-trichloroethyl, 2-chloroethyl, 2-bromoethyl, 2-fluoroethyl, 2-iodoethyl and 2,2-dibromoethyl esters; alkoxymethyl esters where the alkoxy part is preferably C₁-C₄, e.g. the methoxymethyl, ethoxymethyl, propoxymethyl, isopropoxymethyl and butoxymethyl esters; aliphatic acyloxyalkyl esters (particularly the acyloxymethyl and acyloxyethyl esters), such as the acetoxymethyl, propionyloxymethyl, butyryloxymethyl, pivaloyloxymethyl, 1-acetoxyethyl, 1-propionyloxyethyl, 1-butyryloxyethyl and 1-pivaloyloxyethyl esters; (C₁-C₄ alkyl)oxycarbonyloxyethyl esters, such as the 1-methoxycarbonyloxyethyl, 1-ethoxycarbonyloxyethyl, 1-propoxycarbonyloxyethyl, 1-isopropoxycarbonyloxyethyl, 1-butoxycarbonyloxyethyl and 1-isobutoxycarbonyloxyethyl esters; heterocyclic esters, such as the phthalidyl esters; heterocyclyl-methyl esters (in which the heterocyclic group may be as defined for R⁹ or may have up to 3 of said nitrogen, oxygen or sulphur hetero-atoms and may be unsubstituted or have up to 3 C₁-C₄ alkyl, C₁-C₄ alkoxy, halogen or oxygen substituents) for example the (5-methyl-2-oxo-1,3-dioxolen-4-yl)methyl esters; and esters which are easily hydrolized in vivo, a class which includes some of the esters of classes mentioned above [e.g. the aliphatic acyloxyalkyl esters, the lower alkoxycarbonyloxyethyl esters, the (5-methyl-2-oxo-1,3-dioxolen-4-yl)methyl esters and the phthalidyl esters].

There is no particular limitation on the nature of the cations employed to form salts of the compounds of the invention, provided that, where they are intended for therapeutic use, they are pharmaceutically acceptable. Again, if they are to be used for non-therapeutic purposes (e.g. as intermediates), even this restriction does not apply. Preferred salts include salts with alkali metals (such as sodium or potassium) or with alkaline earth metals (such as calcium).

The compounds of the invention will also form acid addition salts. The nature of the acid employed to form such salts is not critical, provided that, where they are intended for therapeutic use, they are pharmaceutically acceptable. Again, if they are to be used for non-therapeutic purposes (e.g. as intermediates), even this restriction does not apply. Examples of such acids include: inorganic acids, such as hydrochloric acid, sulphuric acid and phosphoric acid; organic carboxylic acids, such as acetic acid, oxalic acid, maleic acid, succinic acid, citric acid, tartaric acid, fumaric acid, lauric acid, stearic acid and palmitic acid; and such organic sulphonic acids as methanesulphonic acid, benzenesulphonic acid and p-toluenesulphonic acid.

The compounds of the present invention have a number of asymmetric carbon atoms in their molecules and can, therefore, exist in the form of various stereoisomers. The present invention includes both the individual isolated isomers as well as mixtures of these isomers. Griseolic acid, being a natural product, is a single isomer, in which both the 2′ and 7′ carbon atoms are in the R configuration; compounds prepared from griseolic acid may retain the same configuration or may have the inverted configuration at one or more of the asymmetric carbon atoms. For example, when R¹ represents a group or atom other than hydrogen, the configuration of the compounds at the 2′-position may be α or β.
When R² represents a group or atom other than hydrogen, the configuration at the 7′-position may be RS, R or S.

Preferred classes of compound of the present invention are:
1. Compounds of formula (I), in which:
   R¹ and R² are the same or different and each represents a hydrogen atom, a halogen atom or a group of formula -OR^{9a},
   where R^{9a} represents a hydrogen atom, an alkoxycarbonyl group, an alkenyloxycarbonyl group, an aralkyloxycarbonyl group, a C₁-C₂₀ aliphatic carboxylic acyl group or a carbocyclic aromatic carboxylic acyl group,
   and their salts and esters.
2. Compounds as defined in 1 above, in which:
   R³ and R⁴ are the same or different and each represents a carboxy group, a carbamoyl group, a C₂-C₅ alkoxycarbonyl group, a (5-methyl-2-oxo-1,3-dioxolen-4-yl)methoxycarbonyl group, a phthalidyloxycarbonyl group or a C₂-C₅ alkoxycarbonyl group having at least one substituent selected from aryl groups, C₁-C₆ aliphatic carboxylic acyloxy groups and C₁-C₄ alkoxycarbonyloxy groups.
7. Compounds of formula (I), in which:
   R¹ and R² are the same or different and each represents a hydrogen atom, a halogen atom, a hydroxy group, an unsubstituted C₁-C₄ aliphatic acyloxy group or an aromatic acyloxy group in which the aromatic part is a C₆-C₁₀ carbocyclic aryl group which is unsubstituted or has from 1 to 3 substituents selected from nitro groups, halogen atoms, C₁-C₄ alkyl groups, C₁-C₄ alkoxy groups, amino groups, hydroxy groups and cyano groups.
8. Compounds as defined in 7 above, in which:
   R³ represents a carboxy group, a C₂-C₅ alkoxycarbonyl group, a (5-methyl-2-oxo-1,3-dioxolen-4-yl)methoxycarbonyl group, a phthalidyloxycarbonyl group or a C₂-C₅ alkoxycarbonyl group having 1 or 2 substituents selected from C₂-C₅ alkanoyloxy groups, C₁-C₄ alkoxycarbonyloxy groups and C₆-C₁₀ carbocyclic aryl groups which are unsubstituted or have from 1 to 3 substituents selected from nitro groups, halogen atoms, C₁-C₄ alkyl groups, C₁-C₄ alkoxy groups, amino groups, hydroxy groups and cyano groups; and
   R⁴ represents a carbamoyl group, or any one of the groups defined above for R³.
13. Compounds of formula (I), in which:
   R¹ and R² are the same or different and each represents a hydrogen atom, a halogen atom or a hydroxy group.
14. Compounds as defined in 13 above, where:
   R³ and R⁴ are as defined in 8 above.

Examples of compounds of the present invention are shown in the following formulae (I-8), in which the substituents are as defined in Table 8. Where appropriate, the compounds of the invention are hereinafter referred to by the numbers assigned to them in the Table.

In the Table, the abbreviations used have the following meanings:
- Ac: acetyl
- Boz: benzoyl
- Bu: butyl
- Bz: benzyl
- Bzh: benzhydryl
- Dox: (5-methyl-2-oxo-1,3-dioxolen-4-yl)methyl.
- Et: ethyl
- Hx: hexyl
- Me: methyl
- Piv: pivaloyl
- Pn: pentyl

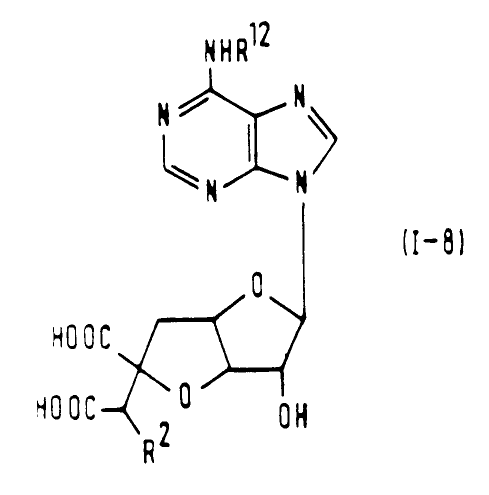

**Table 8**

| Cpd No. | R² | R¹² |
|---|---|---|
| 253 | OH | Me |
| 254 | OH | Et |
| 255 | OH | Pr |
| 256 | OH | Bu |
| 257 | OH | Pn |
| 258 | H | Me |
| 259 | H | Et |
| 260 | H | Pr |
| 261 | H | Bu |
| 262 | H | Pn |
| 263 | H | Hx |
| 264 | OH | Hx |

The compounds of the invention may be prepared as illustrated in the following reaction schemes: 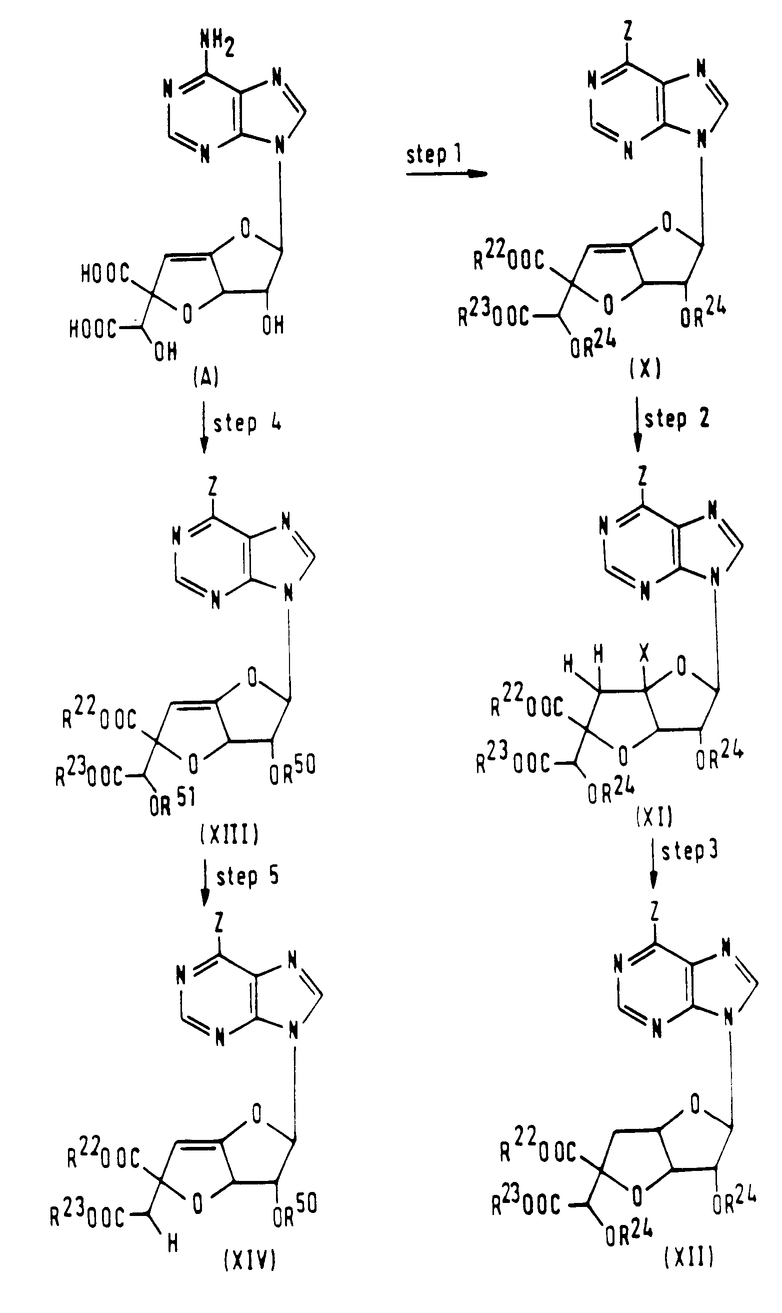 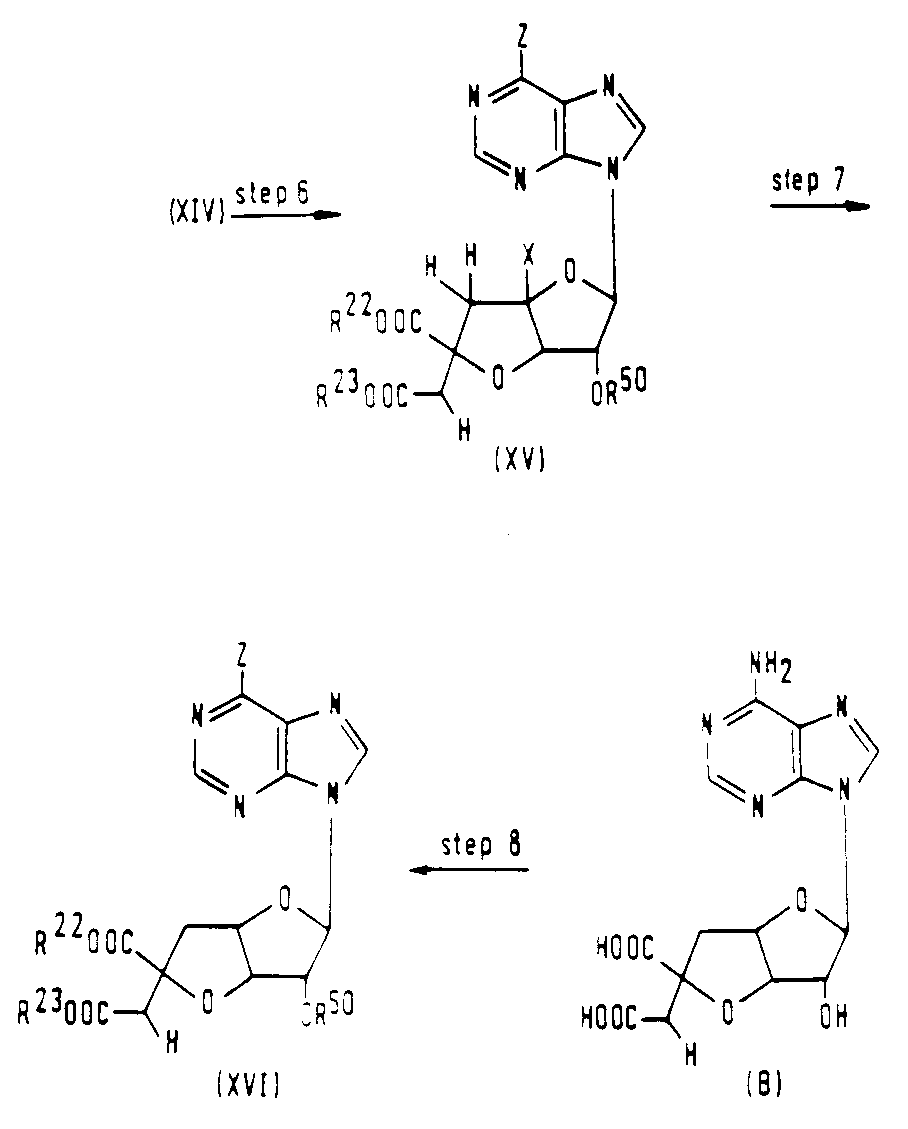 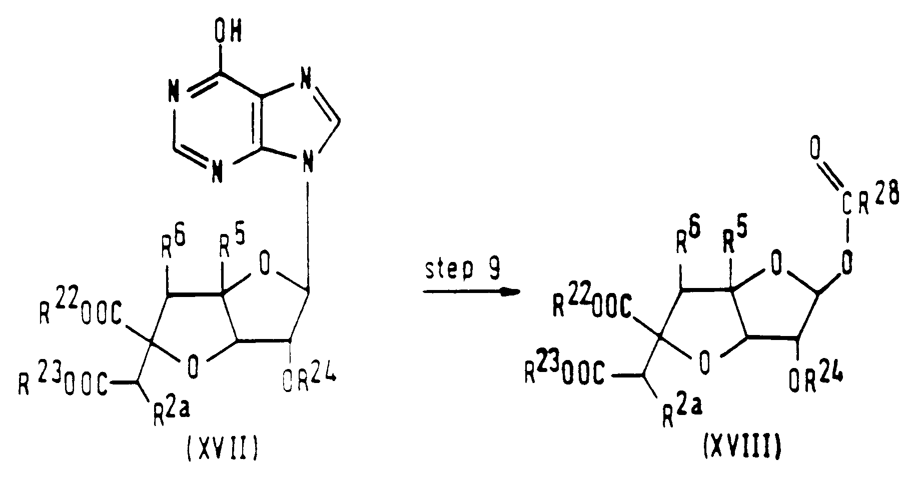 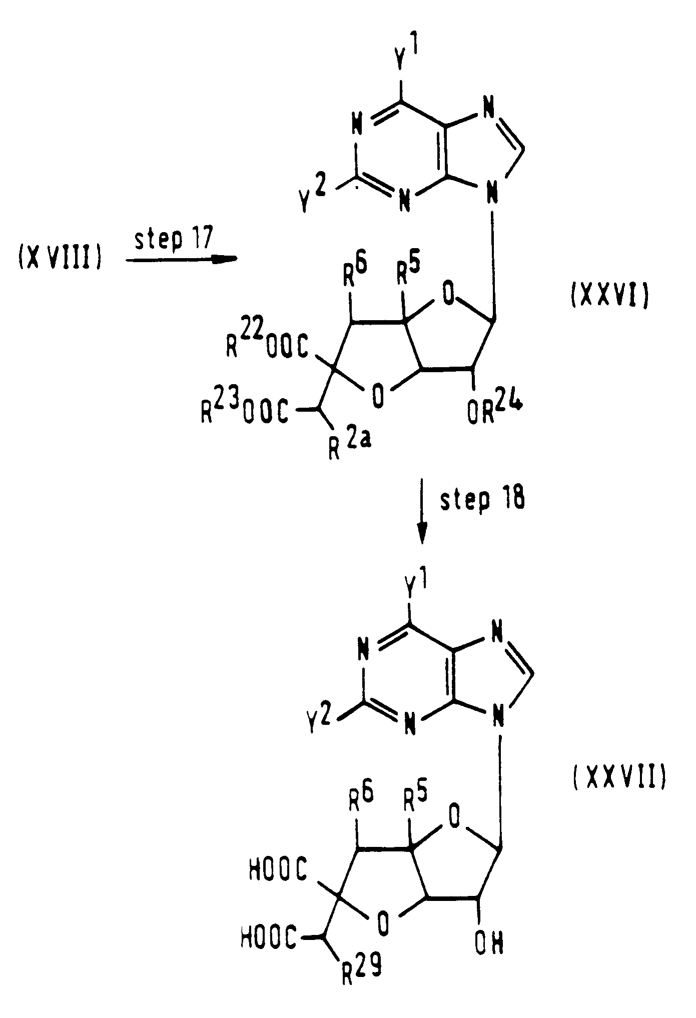 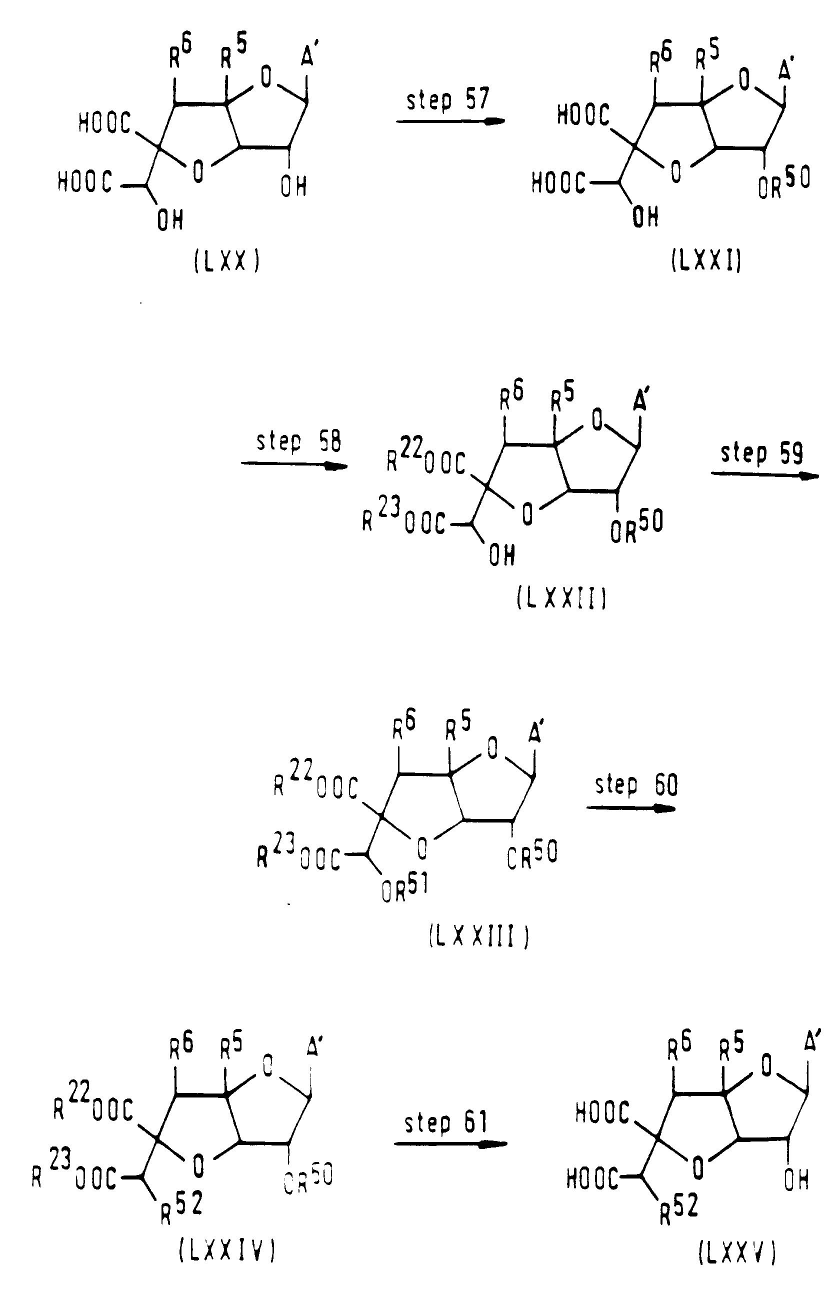 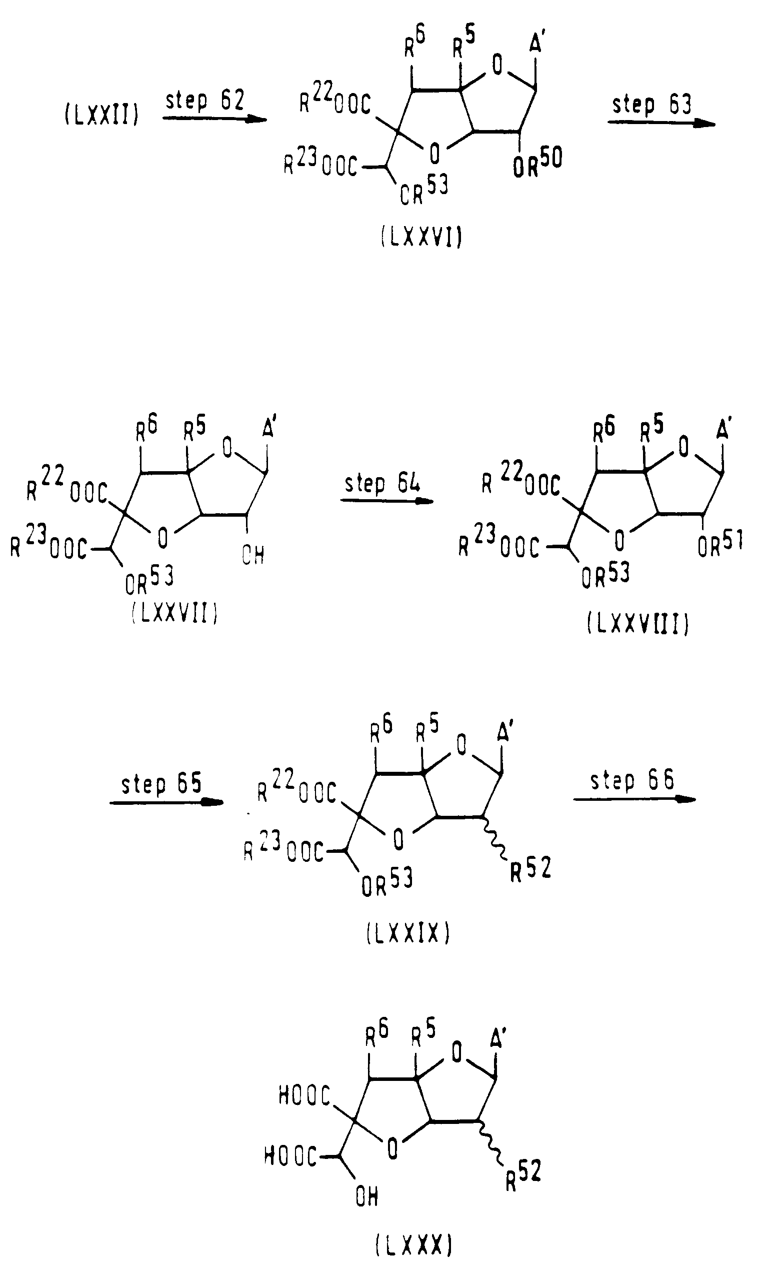 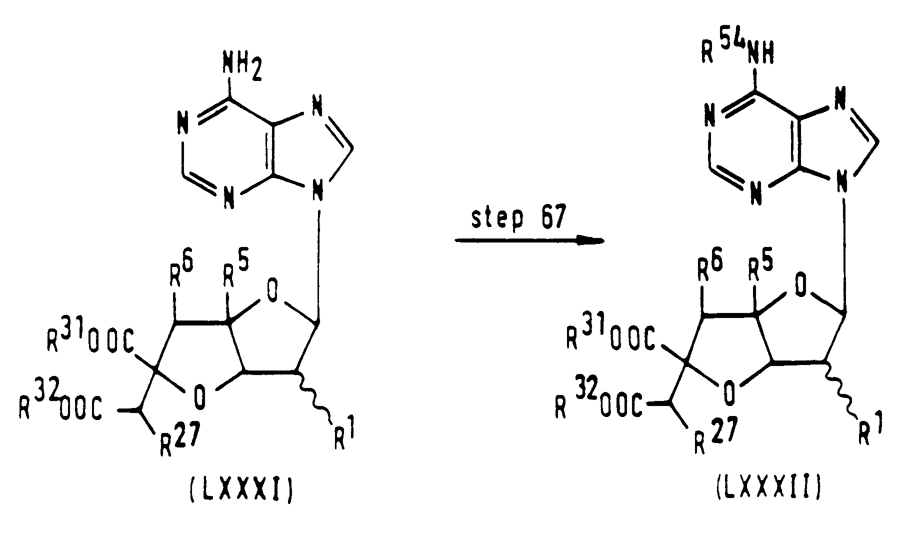
In the above formulae:
R¹-R⁶ are as defined above;
R^{2a} represents a hydrogen atom or a protected hydroxy group;
R²² and R²³ are the same or different and each represents a carboxy-protecting group;
R²⁴ represents a hydroxy-protecting group;
X represents a halogen atom;
R²⁷ represents a hydrogen atom, a hydroxy group or a protected hydroxy group;
R²⁸ represents a lower alkyl group, e.g. any one of the lower alkyl groups defined in relation to R¹²;
A' represents an appropriate purine derivative or analogue;
R²⁹ represents a hydrogen atom or a hydroxy group;
Y¹ and Y² are the same or different and each represents a hydrogen atom, a hydroxy group, a mercapto group, an amino group, a protected amino group or a group of formula -SR²⁸;
R³¹ and R³² are the same or different and each represents a hydrogen atom or a carboxy-protecting group;
Z represents a hydroxy group or an amino group;
R⁵⁰ represents an aliphatic acyl group, an aromatic acyl group or a trialkylsilyl group;
R⁵¹ represents a C₁-C₆ alkylsulphonyl group, a C₁-C₆ fluoroalkylsulphonyl group or an arylsulphonyl group;
R⁵² represents a hydrogen atom or a halogen atom;
R⁵³ represents a tetrahydropyranyl group or a trialkylsilyl group (e.g. as defined in relation to R⁹); and
R⁵⁴ represents a C₁-C₆ alkyl group.

In these reaction schemes, the starting materials are either griseolic acid, which has the formula (A) or dihydrodesoxygriseolic acid, which has the formula (B), given below: 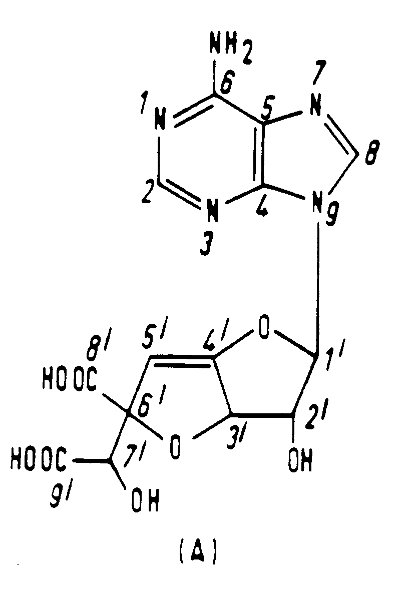 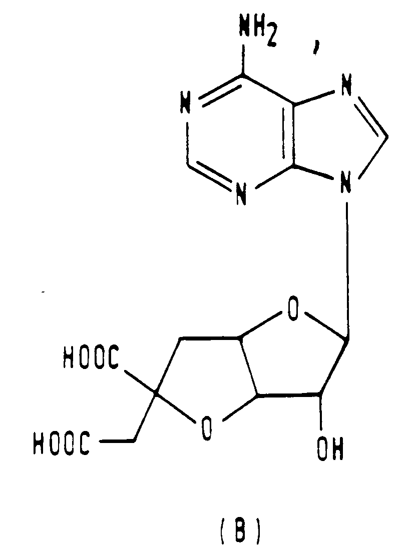
In the above formula (A), representing griseolic acid, we have indicated, for the avoidance of doubt, the numbering system employed throughout this specification.

As already described above, griseolic acid is a known compound disclosed, for example, in European Patent Specification No. 29,329 or in US Patent Specification No. 4,460,765. Dihydrodesoxygriseolic acid was disclosed in European Patent Publication No. 0162715, published after the priority hereof. Both griseolic acid and dihydrodesoxygriseolic acid may be produced by cultivating suitable microorganisms of the genus Streptomyces, especially Streptomyces griseoaurantiacus SANK 63479 (deposited on 9th October 1979 at the Fermentation Research Institute, Agency of Industrial Science and Technology, Japan, whence it is available under the Accession No. FERM-P5223, and on 22nd October 1980 at the Agricultural Research Service, Peoria, U.S.A., whence it is available under the Accession No. NRRL 12314). Full details of the characteristics of Streptomyces griseoaurantiacus SANK 63479 are given in European Patent Publication No. 29,329A and in US Patent Specification No. 4,460,765.

### Step 1

In this step, griseolic acid (A) is reacted to protect its hydroxy and carboxy groups. The nature of the reaction employed will depend upon the nature of the protecting groups which are desired and the following reactions are given purely for illustrative purposes. It will, of course, be understood that any reaction known in the art for protecting carboxy groups or hydroxy groups may equally be employed in this step.

In order to protect the carboxy groups, the griseolic acid (A) is preferably reacted with a diazo compound, for example diazomethane or diphenyldiazomethane, or with a triazene compound, particularly a p-tolyltriazene derivative, such as N-methyl-p-tolyltriazene. The reaction is preferably effected in the presence of a solvent, the nature of which is not critical, provided that it has no adverse effect upon the reaction and provided that the starting materials can be dissolved in the solvent, at least to some extent. Suitable solvents include, for example: ketones, such as acetone; ethers, such as tetrahydrofuran; amides, such as dimethylformamide; and mixtures of water with one or more of the above organic solvents. The reaction will take place over a wide range of temperatures and the particular reaction temperature chosen is not critical, although we generally prefer to carry out the reaction at a temperature of from -20°C to +50°C. The time required for the reaction will vary, depending upon many factors, notably the nature of the starting materials and the reaction temperature; however, for example, at room temperature, the reaction will normally require a period of from 1 to 24 hours.

Before or after the protection of the carboxy groups, the hydroxy groups of griseolic acid are also protected. This may be achieved, for example, by reacting the griseolic acid or carboxy-protected griseolic acid with an acid halide, such as acetyl chloride or benzoyl bromide, or with an acid anhydride, such as acetic anhydride, in the presence of a base. The reaction is preferably effected in the presence of a solvent, the nature of which is not critical, provided that it has no adverse effect upon the reaction. In general, we prefer to use pyridine, which also serves as the base. The reaction will take place over a wide range of temperatures and the particular reaction temperature chosen is not critical; however, we generally prefer to carry out the reaction at a temperature within the range from -20°C to room temperature. The time required for the reaction may vary widely, depending upon many factors, notably the nature of the reagents and the reaction temperature; however, at temperatures within the range suggested, a period of from 1 to 15 hours will normally suffice.

If desired, the amino group at the 6-position of the griseolic acid is also converted to a hydroxy group. This reaction is preferably effected by reacting the griseolic acid or protected griseolic acid with a salt of nitrous acid, such as sodium nitrite, in the presence of acetic acid. The reaction is preferably effected in the presence of a solvent, the nature of which is not critical and, accordingly, we normally prefer to employ aqueous acetic acid. If the starting material is only slightly soluble, an acetic acid buffer of pH about 4 may be used. The reaction will take place over a wide range of temperatures, although we generally find it convenient to carry out the reaction at about room temperature. The time required for the reaction may vary widely, depending upon many factors, notably the nature of the reagents and the reaction temperature; however, at the suggested temperature, a period of from 15 to 50 hours will normally suffice.

### Step 2

In this step, a hydrogen halide is added across the double bond of the griseolic acid derivative of formula (X), to give the compound of formula (XI). The nature of the hydrogen halide H-X employed in this reaction will depend upon the nature of the halogen atom X which it is desired to introduce, but we generally prefer to use hydrochloric acid, hydrobromic acid or hydroiodic acid. The reaction is preferably effected in the presence of a solvent, the nature of which is not critical, provided that it has no adverse effect upon the reaction and that it can dissolve the starting materials, at least to some degree. An example of a suitable solvent is an organic acid, such as acetic acid. The reaction will take place over a wide range of temperatures, for example from 0°C to 100°C, although we generally find it convenient either to carry out the reaction at a temperature in the range from 0°C to room temperature or with heating at a temperature in the range from 80°C to 100°C. The time required for the reaction may vary widely, depending upon many factors, notably the reaction temperature and the natures of the solvent and reagents, but a period of from 1 to 72 hours will normally suffice.

### Step 3

In this step, the halogen atom X at the 4′-position of the compound of formula (XI), prepared in Step 2, is removed by reduction. The reducing agent is preferably either a tri-substituted tin hydride, such as tributyltin hydride, in an aromatic hydrocarbon solvent, such as benzene, or zinc powder, in which case the solvent is preferably a lower aliphatic acid, such as acetic acid, or alcohol, such as methanol or ethanol. When the tri-substituted tin hydride is used as the reducing agent, the reaction is preferably carried out at about the boiling temperature of the solvent and the period required for the reaction is generally from 2 to 10 hours. When zinc powder is the reducing agent, the reaction is preferably effected at a temperature from room temperature to 100°C and the period required is generally from 2 to 20 hours.

### Step 4

In this step, griseolic acid, of formula (A), is converted to a derivative thereof of formula (XIII).

The hydroxy group at the 2′-position may be protected by reaction with an acylating agent, e.g. as also described in Step 1, to introduce the acyl group R⁵⁰, and then carboxy groups of the griseolic acid may be protected. If desired, the 6-amino group of the griseolic acid may be converted to a hydroxy group, employing the appropriate reactions described in Step 1, and this conversion may take place before or after the two above-mentioned protecting steps.

The hydroxy group at the 7′-position is converted to a sulphonyloxy group -OR⁵¹ by reacting the compound with a sulphonylating agent, for example a lower alkylsulphonyl halide (such as methanesulphonyl chloride), an arylsulphonyl halide (such as p-toluenesulphonyl chloride) or a fluorinated lower alkylsulphonyl halide (such as trifluoromethanesulphonyl chloride). The reaction is preferably effected in the presence of an acid-binding agent, whose function is to remove from the reaction medium the hydrogen halide liberated by this reaction. Suitable acid-binding agents include pyridine and dimethylaminopyridine. The reaction is preferably effected in the presence of a solvent, the nature of which is not critical, provided that it does not have any adverse effect upon the reaction. Suitable solvents include halogenated hydrocarbons, especially halogenated aliphatic hydrocarbons, such as methylene chloride or chloroform. The reaction will take place over a wide range of temperatures and there is no particular limitation on the precise temperature chosen; we generally find it convenient to carry out the reaction at a temperature within the range from -10°C to room temperature. The time required for the reaction will vary widely, depending upon many factors, notably the reaction temperature and the nature of the reagents; however, a period of from 1 to 20 hours will normally suffice.

### Step 5

In this step, the sulphonyloxy group at the 7′-position of the compound of formula (XIII) is replaced by a halogen atom (by reaction with an anhydrous lithium halide in an acid amide such as dimethylformamide) and then by a hydrogen atom (by a reducing agent).

The former reaction is preferably carried out by the same method as described in the Step 60 hereafter.

The reducing agent is preferably zinc in aqueous acetic acid, in which case the aqueous acetic acid itself may serve as the reaction solvent. The reaction will take place over a wide range of temperatures, for example from 0°C to 150°C and the time required for the reaction, which may vary widely, is generally from 1 to 10 hours.

### Steps 6 and 7

In these steps, the compound of formula (XIV), prepared as described in Step 5, is first reacted with a hydrogen halide to prepare a compound of formula (XV), and then this compound of formula (XV) is subjected to reduction to give the compound of formula (XVI). The reactions involved in these steps are precisely the same as those described above in relation to Steps 2 and 3 and may be carried out employing the same reagents and reaction conditions.

### Step 8

In this step, dihydrodesoxygriseolic acid of formula (B) has its carboxy and hydroxy groups protected and optionally has its 6-amino group converted to a hydroxy group. The reactions involved are precisely the same as described in relation to Step 1 and may be carried out employing the same reagents and under the same reaction conditions.

### Step 9

The starting material for this step, the compound of formula (XVII), may be any of the compounds of formulae (XII) or (XVI), prepared as described above. In this step, the nucleic acid base at the 1′-position is converted to an alkanoyloxy group by reacting the compound of formula (XVII) with (i) sulphuric acid or trifluoromethanesulphonic acid, (ii) a lower carboxylic acid and (iii) an anhydride thereof. The reaction is preferably effected in the presence of a solvent, the nature of which is not critical, provided that it has no adverse effect upon the reaction and that the reagents will dissolve in it, at least to some degree. Lower aliphatic carboxylic acids, which also serve as a reagent, are the preferred solvents. The reaction will take place over a wide range of temperatures, for example from 0°C to 100°C; in general, we prefer to carry out the reaction either at a temperature from 0°C to room temperature or, with heating, at a temperature from 80°C to 100°C. The time required for the reaction will vary, depending upon many factors, notably the reaction solvent and the reaction temperature, but a period of from 1 to 72 hours will normally suffice.

### Step 17

In this step, the sugar derivative of formula (XVIII) is subjected to a glycosidation reaction with a trimethylsilylated nucleic acid base, to give a compound of formula (XXVI). The reagents and reaction conditions are essentially as described above in relation to Step 10.

### Step 18

In this step, the compound of formula (XXVI) prepared in Step 17, is subjected to a reaction to remove protecting groups and give the compound of formula (XXVII). The reactions involved are essentially as described in Step 11 and may be carried out employing the same reagents and reaction conditions.

### Step 57

In this step, only the hydroxy group at the 2'-position of the sugar part of the griseolic acid derivative of formula (LXX), which can have been prepared by any of the processes described above, is acylated. This can be achieved either by:
(i) slowly adding a base (such as sodium hydroxide) to the griseolic acid derivative (LXX), followed by the addition of an acylating agent (any of those described in relation to previous acylation steps, but particularly an aromatic acyl halide, such as benzoyl chloride) to the reaction solution, whilst keeping its pH at a value of from 10 to 13; or
(ii) dissolving the griseolic acid derivative (LXX) in a buffer solution of pH from 10 to 13, followed by the addition of an acylating agent.

The reaction is preferably effected in the presence of a solvent, the nature of which is not critical, provided that it has no adverse effect upon the reaction and that it can dissolve the reagents at least to some degree. A mixture of water and a water-immiscible solvent (particularly an ester, such as ethyl acetate) is preferred. The reaction will take place over a wide range of temperatures, for example from -20°C to +50°C. The time required for the reaction may vary widely, depending upon many factors, notably the nature of the reagents and the reaction temperature; however, a period of from 1 to 10 hours will normally suffice.

### Step 58

In this step, the free carboxy groups of the griseolic acid derivative of formula (LXXI) (prepared as described in Step 57) are protected by a procedure similar to the esterification described in Step 1.

### Step 59

In this step, the compound of formula (LXXII), prepared as described in Step 58, is converted to a compound of formula (LXXIII) by sulphonylation. The reaction is achieved by reacting the compound of formula (LXXII) with a lower alkylsulphonyl halide (such as methanesulphonyl chloride), an arylsulphonyl halide (such as p-toluenesulphonyl chloride) or a fluorinated lower alkylsulphonyl halide (such as trifluoromethanesulphonyl chloride) and with an acid-binding agent, such as pyridine or dimethylaminopyridine. The reaction is preferably effected in the presence of a solvent, the nature of which is not critical, provided that it has no adverse effect upon the reaction. Suitable solvents include: halogenated hydrocarbons, particularly halogenated aliphatic hydrocarbons, such as methylene chloride or chloroform. The reaction will take place over a wide range of temperatures, although we generally prefer a relatively low temperature, e.g. from -10°C to room temperature. The time required for the reaction may vary, depending upon many factors, notably the nature of the reagents and the reaction temperature; however, a period of from 1 to 20 hours will normally suffice.

### Step 60

In this step, the sulphonyloxy group at the 7′-position of the compound of formula (LXXIII) is replaced by a halogen atom (by reaction with an anhydrous lithium halide in an acid amide such as dimethylformamide) and/or by a hydrogen atom (by reduction using zinc/aqueous acetic acid as described in Step 5). The former reaction is preferably effected in the presence of a solvent, the nature of which is not critical, provided that it has no adverse effect upon the reaction. Suitable solvents include such polar solvents as dimethylformamide, dimethyl sulphoxide, triethyl phosphate or hexamethylphosphoric triamide. The reaction will take place over a wide range of temperatures, for example from 0 to 150°C. The time required for the reaction may vary widely, depending upon many factors, but a period of from 1 to 10 hours will normally suffice.

### Step 61

In this step, the protecting groups are removed from the compound of formula (LXXIV) by methods appropriate to the nature of the protecting group. When, for example, a pyranyl group is employed as the hydroxy-protecting group, it may be removed by treatment with an acid, such as acetic acid or p-toluenesulphonic acid, preferably with pyridine p-toluenesulphonate, in a mixed solvent comprising a lower alkanol (such as ethanol) and a halogenated hydrocarbon (such as methylene chloride). When a trialkylsilyl group is employed as the hydroxy-protecting group, it can be removed by treatment with a compound generating a fluorine anion, such as tetrabutylammonium fluoride, with an ether (such as tetrahydrofuran) serving as solvent. The reaction will take place over a wide range of temperatures, for example from room temperature to 100°C. The time required for the reaction may vary widely, depending upon many factors, but a period of from 5 to 20 hours will normally suffice. Carboxy-protecting groups and/or other hydroxy-protecting groups may be removed using techniques standard in the art.

### Step 62

In this step, the hydroxy group at the 7′-position of the compound of formula (LXXII) (prepared as described in Step 58) is protected by various methods appropriate to the particular protecting group which it is desired to introduce. If it is desired to protect the group by means of a pyranyl group, then the compound (LXXII) is allowed to react with a suitable pyran derivative, for example 3,4-dihydro-α-pyran, in the presence of an acid catalyst, such as hydrochloric acid. If the group is to be protected by means of a lower trialkylsilyl group, then the compound (LXXII) is allowed to react with a trialkylsilyl halide, such as dimethyl-t-butylsilyl chloride, and with imidazole. The reactions are preferably effected in the presence of a solvent, the nature of which is not critical, provided that it has no adverse effect upon the reaction. Suitable solvents include, for example: halogenated hydrocarbons, particularly halogenated aliphatic hydrocarbons, such as chloroform; esters, such as ethyl acetate; ethers, such as dioxane; and acid amides, such as dimethylformamide. The reaction will take place over a wide range of temperatures and the particular temperature chosen is not critical to the invention. We normally find it convenient to carry out the reaction at about room temperature. The time required for the reaction may vary widely, depending upon many factors, notably the natures of the reagents and solvents and the reaction temperature; however, under the conditions suggested above, a period of from 1 to 30 hours will normally suffice.

### Step 63

In this step, the 2′-hydroxy group is deprotected by removing the acyl or silyl group R⁵⁰ from the compound of formula (LXXVI) to give the compound of formula (LXXVII). The reaction is preferably effected by contacting the protected compound with an aqueous solution of an alkali metal hydroxide, such as a 1N aqueous solution of sodium hydroxide, or with 20% v/v methanolic ammonia. The reaction will take place over a wide range of temperatures, for example from -20 to +50°C. The time required for the reaction may vary widely, depending upon many factors, notably the nature of the reagents and the reaction temperature. However, under the conditions suggested, a period of from 10 minutes to 3 hours will normally suffice.

### Step 64

In this step, the compound of formula (LXXVII) is sulphonylated by a process analogous to that described in Step 59, and the reaction may be carried out under the same reaction conditions and employing the same reagents, to give the compound of formula (LXXVIII).

### Step 65

In this step, the sulphonyloxy group at the 2′-position of the compound of formula (LXXVIII) is replaced by a halogen atom or by a hydrogen atom. The reactions involved are similar to those described in Step 60 and may be carried out employing the same reagents and under the same reaction conditions.

### Step 66

In this step, the compound of formula (LXXIX), prepared as described in Step 65, is deprotected to give the compound of formula (LXXX). The reactions involved are similar to those described in Step 61 and may be carried out employing the same reagents and under the same reaction conditions.

Since each of the substitution reactions of Steps 60 and 65 involves a Walden inversion, it affords a compound of inverted steric configuration, as compared with the initial compound. Any compound having the inverse configuration of that of the compound obtained above (i.e. the naturally occuring configuration) can be prepared, if desired, as follows: in the procedure of Step 60 or Step 65, a lower alkanoyloxy group is introduced into the starting material as R⁵²; this lower alkanoyloxy group is then removed by the procedure described in Step 61; and then the resulting compound is subjected again to the sequence of steps from Step 59 or to the sequence steps from Step 64, respectively.

### Step 67

In the first part of Step 67, the griseolic acid or derivative thereof of formula (LXXXI) is subjected to alkylation or aralkylation with an alkylating or aralkylating agent in an inert solvent. The nature of the solvent is not particularly critical, provided that it has no adverse effect upon the reaction. Examples of suitable solvents include: alcohols, such as methanol, ethanol, isopropanol, butanol and t-butanol; ethers, such as diethyl ether, tetrahydrofuran, dioxane or ethylene glycol dimethyl ether; nitriles, such as acetonitrile; amides such as dimethylformamide, dimethylacetamide or hexamethylphosphoric triamide; and sulphoxides, such as dimethyl sulphoxide. The preferred solvents are amides or sulphoxides.

The reaction will take place over a wide range of temperatures, but we prefer to carry out the reaction at a temperature of from 0°C to 100°C, more preferably from room temperature to 70°C.

The time required for the reaction may vary widely, depending upon many factors, notably the reaction temperature and the natures of the solvents and reagents employed. In general, a period of from 30 minutes to 10 days will suffice. If, for example, the reaction is carried out at room temperature, it is generally complete within from 1 to 7 days; on the other hand, at 70°C, it will normally be complete within from 1 to 20 hours.

The intermediate product thus produced may be obtained from the reaction mixture by evaporating off the solvent under reduced pressure and then the product may be subjected to the next part of the Step without further isolation, in the same reaction vessel. Alternatively, if desired, the intermediate may be isolated by conventional means before being subjected to the next part of the Step.

In the second part of Step 67, the intermediate compound is subjected to a ring-opening, rearrangement and ring-closure reaction involving the pyrimidine ring and the free amino group.

In this step, the residue obtained from the alkylation or aralkylation reaction is dissolved or suspended in a suitable solvent and the pH of the resulting solution or suspension is adjusted or maintained at a value not less than 4, to effect the aforesaid ring-opening, rearrangement and ring-closure reactions. The pH value employed for three reactions is more preferably at least 5 and still more preferably at least 7.

Maintenance of the chosen pH value may be achieved, for example, either (1) by conducting the reactions in a buffer solution previously adjusted to an appropriate pH value or (2) by standing or heating the residue in an excess of an aqueous solution of an alkali metal or alkaline earth metal hydroxide or a solution containing an organic base in water or in a suitable organic solvent.

There is no particular limitation upon the nature of the buffer solution to be employed, provided that it is capable of maintaining an appropriate pH value throughout the reaction. Any conventional buffer solution, for example an acetate, phosphate, borate, ammonium bicarbonate, phthalate or citrate buffer, may be used.

Examples of suitable alkali metal and alkaline earth metal hydroxides which may be used in the aqueous solution include sodium hydroxide, potassium hydroxide, lithium hydroxide and calcium hydroxide. Examples of suitable organic bases include, for example, lower alkylamines, such as monomethylamine, dimethylamine or trimethylamine.

In general, the pH of the reaction solution is preferably maintained within the range from 4 to 12, although higher pH values may also be employed.

There is no particular limitation on the nature of the solvent employed in this reaction, provided that it does not interfere with the reactions. Suitable solvents include, for example: water; alcohols, such as methanol, ethanol or propanol; and other water-miscible solvents, such as acetone, tetrahydrofuran, dioxane, dimethylformamide or dimethyl sulphoxide. A single such solvent or a mixture of any two or more thereof may be employed. In some cases, the organic base may also act as the reaction solvent.

The reaction may take place over a wide range of temperatures, for example from 0°C to 150°C, more preferably from 20°C to 100°C. The temperature chosen may depend upon various factors. For example, heating may be preferable when the reaction is carried out at a pH value within the range from 4 to 10; on the other hand, the reaction will generally proceed satisfactorily at ambient temperature at a pH of 10 or above.

The time required for the reaction may vary widely, depending upon many factors, notably the nature of the substrates, the reaction temperature and the pH and nature of the buffer or other medium used, especially the temperature and pH; however, within the preferred ranges indicated above, a period of from 5 minutes to 50 hours will normally suffice. If desired, the optional steps of Step 27 may then be carried out.

After completion of any of the reactions described above, the desired product of each step may be separated from the reaction mixture by conventional means. For example, the reaction mixture is, if necessary, washed with water, and then the solvent is distilled off under reduced pressure. The residue can be purified by various means, such as recrystallization or the various chromatography techniques, such as column chromatography or preparative thin layer chromatography, to afford the desired compound.

The compounds show a variety of therapeutic uses, for example: in the treatment of cardiovascular problems; as an antiasthmatic agent; as a smooth muscle relaxant; as a psychotropic or neurotropic agent; as an anti-inflammatory agent; in the therapy of cancer; as a treatment for diabetes; as therapeutic agents for various cerebral circulatory disorders, such as cerebral apoplexy sequelae and cerebral infarction sequelae, and as brain metabolism activators, for example for the therapy of senile dementia or traumatic brain infarction. The compounds of the invention may be administered orally or non-orally (for example by subcutaneous or intramuscular injection).

The compounds of the invention may be administered orally in the form of solid preparations which may, if necessary, contain various conventional additives. Such additives include: diluents, such as sugars and cellulose preparations; binders, such as starch, gums and methylcellulose; and disintegrating agents. The dosage will vary depending upon the symptoms, age and body weight of the patient. For example, in the case of an adult human patient, a suitable daily dose would be from 0.1 to 100 mg/kg of the active compound, which may be administered in a single dose or in divided doses.

The preparation of various compounds of the present invention is illustrated in the following Example. The preparation of starting materials is illustrated in the subsequent Preparation.

### Example 1

### N⁶-Methyl-7′-desoxy-4′α,5′-dihydrogriseolic acid

1 ml of methyl iodide was added to a solution of 100 mg of 7′-desoxy-4′α,5′-dihydrogriseolic acid in 20 ml of dimethylformamide, and the mixture was allowed to stand at room temperature for 24 hours in a sealed vessel. The solvent was distilled off under reduced pressure to give a residue. 10 ml each of acetone and toluene were added to the residue and the mixture was concentrated by evaporation under reduced pressure. This operation was repeated twice. A solution of the resulting residue in 20 ml of a 0.5M phosphate buffer of pH 7.0 was stirred for 3 hours under reflux, to give a reaction mixture which was purified by column chromatography using an RP-8 prepacked column (Merck) followed by lyophilizing the main fractions to give 67 mg of the title compound as a white powder.
Ultraviolet Absorption Spectrum (ε) λₘₐₓ:
pH 1.0 262 nm (17700).
H₂O 264 nm (16700).
pH 13 266 nm (17100).
Nuclear Magnetic Resonance Spectrum [(CD₃)₂SO+D₂O] δ ppm:
2.28-2.31 (2H, multiplet);
2.80-3.03 (5H, multiplet);
4.37-4.46 (3H, multiplet);
6.16 (1H, singlet);
8.26 (1H, doublet);
8.28 (1H, singlet).

### PREPARATION

### Dihydrodesoxygriseolic acid

30 litres of a medium having a pH of 7.0 before sterilization and the following composition (percentages are w/v) were prepared:
Glucose 5%
Soybean Meal 1%
Yeast Extract 0.1%
Polypeptone 0.4%
Meat Extract 0.4%
Sodium Chloride 0.25%
Calcium Carbonate 0.5%
Water to 100%
15 litres of this medium were charged into each of two 30 litre jar fermenters, which were then sterilized under pressure at 120°C for 30 minutes. The culture medium was cooled, and then 150 ml (1% by volume) of a culture broth of Streptomyces griseoaurantiacus SANK 63479 (which has previously been incubated in the medium described above by means of a rotatory shaking cultivator at 28°C for 72 hours) were inoculated into each fermenter. Cultivation was then carried out at 28°C for 96 hours under aeration at the rate of 15 litres per minute and with agitation at the rate of 200 rpm.

The two culture broths were then filtered to remove the mycelial cake and the combined filtrates (pH 7.0), in a total volume of 28 litres, were passed through a column of Diaion HP 20 (a trademark for an ion-exchange resin produced by Mitsubishi Chemical Industries Ltd.) and then adsorbed on a column of activated charcoal. This column was washed with water and then the adsorbed material was eluted with a 60:40 by volume mixture of acetone and water. The acetone was evaporated from the resulting solution under reduced pressure and the remaining aqueous solution was concentrated by evaporation under reduced pressure and then lyophilized, to give 150 mg of a crude powder.

This crude powder was dissolved in a small amount of distilled water and then adsorbed on Dowex 1 x 4 (Cl⁻ form, a trademark for an ion-exchange resin produced by the Dow Chemical Company). At this stage, the product was a mixture of griseolic acid and dihydrodesoxygriseolic acid. This mixture was subjected to gradient elution with a sodium chloride gradient to separate the two components and then the eluate was subjected to column chromatography through Sephadex LH-20 (a trademark for a product of Pharmacia Co) and the dihydrodesoxygriseolic acid was eluted with water. The fractions containing this substance were combined and their pH was adjusted to a value of 2.5 by the addition of 1N aqueous hydrochloric acid. The product was then adsorbed on a column of Diaion HP 20, washed with water and then eluted with a 60:40 by volume mixture of acetone and water. The eluate was left to stand overnight at 4°C, whereupon the dihydrodesoxygriseolic acid separated out as plates. These were separated from the liquor, giving a total of 1.87 mg of dihydrodesoxygriseolic acid, as white plates melting at 160°C (with decomposition, accompanied by a brown discolouration). This compound gave a single spot on silica gel thin layer chromatography (silica gel Art. 5715, a product of Merck & Co. Inc.).

## Claims (Claims for the following Contracting State(s): AT)

1. A process for the preparation of compounds of formula (I): 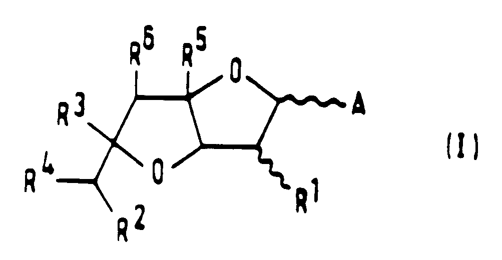 in which:
A represents a group of formula: 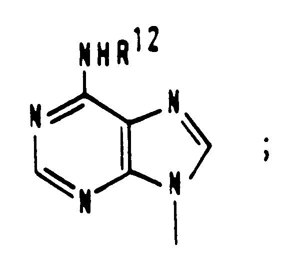 R¹ and R² are the same or different and each represents a hydrogen atom, a halogen atom or a group of formula -OR⁹;
R³ and R⁴ are the same or different and each represents a carbamoyl group or a carboxy group;
R⁵ and R⁶ both represent hydrogen atoms;
R⁹ represents a hydrogen atom, a C₁-C₆ alkyl group, an alkylsulphonyl group, or haloalkylsulphonyl group, an arylsulphonyl group or a hydroxy-protecting group;
R¹² represents a c₁-C₆ alkyl group;
and pharmaceutically acceptable salts and esters thereof;
which process comprises replacing the adenine moiety of griseolic acid or of dihydrodesoxygriseolic acid by another group of formula (A) and/or by replacing any of the groups defined above as R¹, R², R³, R⁴, R⁵, R⁶, R⁹ and R¹² by any other group within the definitions of said groups.

2. A process as claimed in Claim 1, wherein:
R¹ and R² are the same or different and each represents a hydrogen atom, a halogen atom or a group of formula -OR^{9a},
wherein R^{9a} represents a hydrogen atom, an alkoxycarbonyl group, an alkenyloxycarbonyl group, an aralkyloxycarbonyl group, a C₁-C₂₀ aliphatic carboxylic acyl group or a carbocyclic aromatic carboxylic acyl group.

3. A process as claimed in Claim 1 or Claim 2, wherein:
R³ and R⁴ are the same or different and each represents a carboxy group, a carbamoyl group, a C₂-C₅ alkoxycarbonyl group, a (5-methyl-2-oxo-1,3-dioxolen-4-yl)methoxycarbonyl group, a phthalidyloxycarbonyl group or a C₂-C₅ alkoxycarbonyl group having at least one substituent selected from aryl groups, C₁-C₆ aliphatic carboxylic acyloxy groups and C₁-C₄ alkoxycarbonyloxy groups.

4. A process as claimed in Claim 1, wherein:
R¹ and R² are the same of different and each represents a hydrogen atom, a halogen atom, a hydroxy group, an unsubstituted C₁-C₄ aliphatic acyloxy group or an aromatic acyloxy group in which the aromatic part is a C₆-C₁₀ carbocyclic aryl group which is unsubstituted or has from 1 to 3 substituents selected from nitro groups, halogen atoms, C₁-C₄ alkyl groups, C₁-C₄ alkoxy groups, amino groups, hydroxy groups and cyano groups.

5. A process as claimed in Claim 4, wherein:
R³ represents a carboxy group, a C₂-C₅ alkoxycarbonyl group, a (5-methyl-2-oxo-1,3-dioxolen -4-yl)methoxycarbonyl group, a phthalidyloxycarbonyl group or a C₂-C₅ alkoxycarbonyl group having 1 or 2 substituents selected from C₂-C₅ alkanoyloxy groups, C₁-C₄ alkoxycarbonyloxy groups and C₆-C₁₀ carbocyclic aryl groups which are unsubstituted or have from 1 to 3 substituents selected from nitro groups, halogen atoms, C₁-C₄ alkyl groups, C₁-C₄ alkoxy groups, amino groups, hydroxy groups and cyano groups; and
R⁴ represents a carbamoyl group, or any one of the groups defined above for R³.

6. A process as claimed in Claim 1, wherein:
R¹ and R² are the same or different and each represents a hydrogen atom, a halogen atom or a hydroxy group.

7. A process as claimed in Claim 6, wherein:
R³ represents a carboxy group, a C₂-C₅ alkoxycarbonyl group, a (5-methyl-2-oxo-1, 3-dioxolen -4-yl)methoxycarbonyl group, a phthalidyloxycarbonyl group or a C₂-C₅ alkoxycarbonyl group having 1 or 2 substituents selected from C₂-C₅ alkanoyloxy groups, C₁-C₄ alkoxycarbonyloxy groups and C₆-C₁₀ carbocyclic aryl groups which are unsubstituted or have from 1 to 3 substituents selected from nitro groups, halogen atoms, C₁-C₄ alkyl groups, C₁-C₄ alkoxy groups, amino groups, hydroxy groups and cyano groups; and
R⁴ represents a carbamoyl group, or any one of the groups defined above for R³.

## Claims (Claims for the following Contracting State(s): BE, CH, LI, DE, FR, GB, IT, LU, NL, SE)

1. Compounds of formula (I): 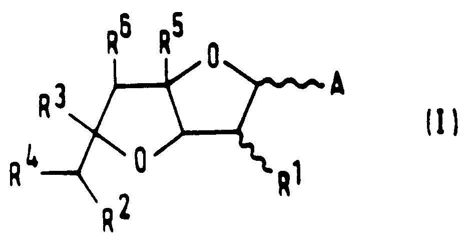 in which:
A represents a group of formula: 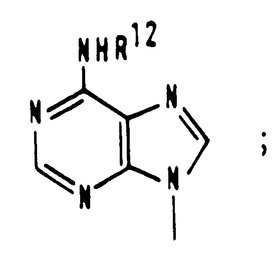 R¹ and R² are the same or different and each represents a hydrogen atom, a halogen atom or a group of formula -OR⁹;
R³ and R⁴ are the same or different and each represents a carbamoyl group or a carboxy group;
R⁵ and R⁶ both represent hydrogen atoms;
R⁹ represents a hydrogen atom, a C₁-C₆ alkyl group, an alkylsulphonyl group, a haloalkylsulphonyl group, an arylsulphonyl group or a hydroxy-protecting group;
R¹² represents a C₁-C₆ alkyl group;
and pharmaceutically acceptable salts and esters thereof.

2. Compounds as claimed in Claim 1, wherein:
R¹ and R² are the same or different and each represents a hydrogen atom, a halogen atom or a group of formula -OR^{9a},
where R^{9a} represents a hydrogen atom, an alkoxycarbonyl group, an alkenyloxycarbonyl group, an aralkyloxycarbonyl group, a C₁-C₂₀ aliphatic carboxylic acyl group or a carbocyclic aromatic carboxylic acyl group.

3. Compounds as claimed in Claim 1 or Claim 2, wherein:
R³ and R⁴ are the same or different and each represents a carboxy group, a carbamoyl group, a C₂-C₅ alkoxycarbonyl group, a (5-methyl-2-oxo-1,3-dioxolen-4-yl)methoxycarbonyl group, a phthalidyloxycarbonyl group or a C₂-C₅ alkoxycarbonyl group having at least one substituent selected from aryl groups, C₁-C₆ aliphatic carboxylic acyloxy groups and C₁-C₄ alkoxycarbonyloxy groups.

4. Compounds as claimed in Claim 1, wherein:
R¹ and R² are the same or different and each represents a hydrogen atom, a halogen atom, a hydroxy group, an unsubstituted C₁-C₄ aliphatic acyloxy group or an aromatic acyloxy group in which the aromatic part is a C₆-C₁₀ carbocyclic aryl group which is unsubstituted or has from 1 to 3 substituents selected from nitro groups, halogen atoms, C₁-C₄ alkyl groups, C₁-C₄ alkoxy groups, amino groups, hydroxy groups and cyano groups.

5. Compounds as claimed in Claim 4, wherein:
R³ represents a carboxy group, a C₂-C₅ alkoxycarbonyl group, a (5-methyl-2-oxo-1,3-dioxolen-4-yl)methoxycarbonyl group, a phthalidyloxycarbonyl group or a C₂-C₅ alkoxycarbonyl group having 1 or 2 substituents selected from C₂-C₅ alkanoyloxy groups, C₁-C₄ alkoxycarbonyloxy groups and C₆-C₁₀ carbocyclic aryl groups which are unsubstituted or have from 1 to 3 substituents selected from nitro groups, halogen atoms, C₁-C₄ alkyl groups, C₁-C₄ alkoxy groups, amino groups, hydroxy groups and cyano groups; and
R⁴ represents a carbamoyl group, or any one of the groups defined above for R³.

6. Compounds as claimed in Claim 1, wherein:
R¹ and R² are the same or different and each represents a hydrogen atom, a halogen atom or a hydroxy group.

7. Compounds as claimed in Claim 6, wherein:
R³ represents a carboxy group, a C₂-C₅ alkoxycarbonyl group, a (5-methyl-2-oxo-1,3-dioxolen-4-yl)methoxycarbonyl group, a phthalidyloxycarbonyl group or a C₂-C₅ alkoxycarbonyl group having 1 or 2 substituents selected from C₂-C₅ alkanoyloxy groups, C₁-C₄ alkoxycarbonyloxy groups and C₆-C₁₀ carbocyclic aryl groups which are unsubstituted or have from 1 to 3 substituents selected from nitro groups, halogen atoms, C₁-C₄ alkyl groups, C₁-C₄ alkoxy groups, amino groups, hydroxy groups and cyano groups; and
R⁴ represents a carbamoyl group, or any one of the groups defined above for R³.

8. A pharmaceutical composition comprising a phosphodiesterase inhibitor in admixture with a pharmaceutically acceptable carrier or diluent, wherein the phosphodiesterase inhibitor is at least one compound as claimed in any one of the preceding Claims.

9. The use in the manufacture of a medicament for the treatment of disorders arising from a phosphodiesterase imbalance of a compound as claimed in any one of Claims 1 to 7.

10. A process for preparing compounds according to any of claims 1 to 7 and pharmaceutically acceptable salts and esters thereof, which process comprises replacing the adenine moiety of griseolic acid or of dihydrodesoxygriseolic acid by another group of formula (A) and/or by replacing any of the groups defined above as R¹, R², R³, R⁴, R⁵, R⁶, R⁹ and R¹² by any other group within the definitions of said groups.

## Patentansprüche (Patentansprüche für folgende(n) Vertragsstaat(en): AT)

1. Verfahren zur Herstellung von Verbindungen der Formel (I): 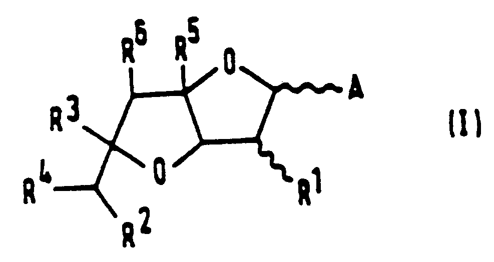 worin
A eine Gruppe der Formel 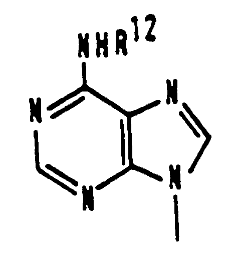 bedeutet,
R¹ und R² gleich oder verschieden sind und jeweils ein Wasserstoffatom, ein Halogenatom oder eine Gruppe der Formel -OR⁹ bedeuten,
R³ und R⁴ gleich oder verschieden sind und jeweils eine Carbamoylgruppe oder eine Carboxygruppe bedeuten,
beide Reste R⁵ und R⁶ Wasserstoffatome darstellen,
R⁹ ein Wasserstoffatom, eine C₁-C₆-Alkylgruppe, eine Alkylsulfonylgruppe, eine Halogenalkylsulfonylgruppe, eine Arylsulfonylgruppe oder eine Hydroxy-Schutzgruppe bedeutet,
R¹² eine C₁-C₆-Alkylgruppe bedeutet,
und von pharmazeutisch geeigneten Salzen und Estern dieser Verbindungen, welches darin besteht, daß die Adenineinheit von Griseolsäure oder Dihydrodesoxygriseolsäure durch eine andere Gruppe der Formel (A) ersetzt wird und/oder, daß irgendeine der vorstehend als R¹, R², R³, R⁴, R⁵, R⁶, R⁹ und R¹² definierten Gruppen durch irgendeine andere Gruppe innerhalb der Definitionen dieser Gruppen ersetzt wird.

2. Verfahren nach Anspruch 1, wobei
R¹ und R² gleich oder verschieden sind und jeweils ein Wasserstoffatom, ein Halogenatom oder eine Gruppe der Formel -OR^{9a} bedeuten,
wobei R^{9a} ein Wasserstoffatom, eine Alkoxycarbonylgruppe, eine Alkenyloxycarbonylgruppe, eine Aralkyloxycarbonylgruppe, eine Acylgruppe einer aliphatischen C₁-C₂₀-Carbonsäure oder eine Acylgruppe einer carbocyclischen aromatischen Carbonsäure darstellt.

3. Verfahren nach Anspruch 1 oder Anspruch 2, wobei
R³ und R⁴ gleich oder verschieden sind und jeweils eine Carboxygruppe, eine Carbamoylgruppe, eine C₂-C₅-Alkoxycarbonylgruppe, eine (5-Methyl-2-oxo-1,3-dioxolen-4-yl)-methoxycarbonylgruppe, eine Phthalidyloxycarbonylgruppe oder eine C₂-C₅-Alkoxycarbonylgruppe, die mindestens einen unter Arylgruppen, Acyloxygruppen von C₁-C₆-aliphatischen Carbonsäuren und C₁-C₄-Alkoxycarbonyloxygruppen ausgewählten Substituenten aufweist, bedeuten.

4. Verfahren nach Anspruch 1, wobei
R¹ und R² gleich oder verschieden sind und jeweils ein Wasserstoffatom, ein Halogenatom, eine Hydroxygruppe, eine unsubstituierte aliphatische C₁-C₄-Acyloxygruppe oder eine aromatische Acyloxygruppe, deren aromatischer Teil eine C₆-C₁₀-carbocyclische Arylgruppe ist, die unsubstituiert ist oder die mindestens 1 bis 3 unter Nitrogruppen, Halogenatomen, C₁-C₄-Alkylgruppen, C₁-C₄-Alkoxygruppen, Aminogruppen, Hydroxygruppen und Cyangruppen ausgewählte Substituenten aufweist, bedeuten.

5. Verfahren nach Anspruch 4, wobei
R³ eine Carboxygruppe, eine C₂-C₅-Alkoxycarbonylgruppe, eine (5-Methyl-2-oxo-1,3-dioxolen-4-yl)-methoxycarbonylgruppe, eine Phthalidyloxycarbonylgruppe oder eine C₂-C₅-Alkoxycarbonylgruppe bedeutet, die 1 oder 2 Substituenten aufweist, die ausgewählt sind unter C₂-C₅-Alkanoyloxygruppen, C₁-C₄-Alkoxycarbonyloxygruppen und carboxyclischen C₆-C₁₀-Arylgruppen, die unsubstituiert sind oder mindestens 1 bis 3 Substituenten aufweisen, die unter Nitrogruppen, Halogenatomen, C₁-C₄-Alkylgruppen, C₁-C₄-Alkoxygruppen, Aminogruppen, Hydroxygruppen und Cyangruppen ausgewählt sind, und
R⁴ eine Carbamoylgruppe oder irgendeine der oben für R³ definierten Gruppen bedeutet.

6. Verfahren nach Anspruch 1, wobei
R¹ und R² gleich oder verschieden sind und jeweils ein Wasserstoffatom, ein Halogenatom oder eine Hydroxygruppe bedeuten.

7. Verfahren nach Anspruch 6, wobei
R³ eine Carboxygruppe, eine C₂-C₅-Alkoxycarbonylgruppe, eine (5-Methyl-2-oxo-1,3-dioxolen-4-yl)-methoxycarbonylgruppe, eine Phthalidyloxycarbonylgruppe oder eine C₂-C₅-Alkoxycarbonylgruppe bedeutet, die 1 oder 2 Substituenten aufweist, die ausgewählt sind unter C₂-C₅-Alkanoyloxygruppen, C₁-C₄-Alkoxycarbonyloxygruppen und carboxyclischen C₆-C₁₀-Arylgruppen, die unsubstituiert sind, oder 1 bis 3 Substituenten, ausgewählt unter Nitrogruppen, Halogenatomen, C₁-C₄-Alkylgruppen, C₁-C₄-Alkoxygruppen, Aminogruppen, Hydroxygruppen und Cyangruppen, aufweisen und
R⁴ eine Carbamoylgruppe oder irgendeine der oben für R³ definierten Gruppen bedeutet.

## Patentansprüche (Patentansprüche für folgende(n) Vertragsstaat(en): BE, CH, LI, DE, FR, GB, IT, LU, NL, SE)

1. Verbindungen der Formel (I): 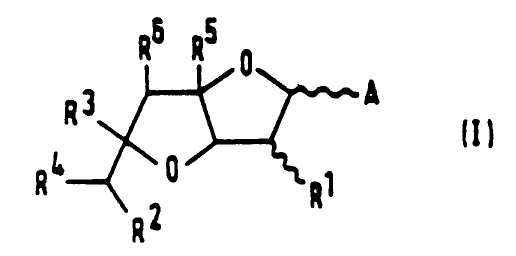 worin
A eine Gruppe der Formel 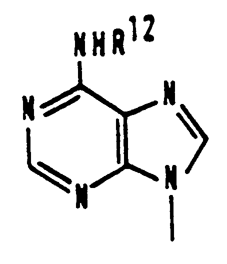 bedeutet,
R¹ und R² gleich oder verschieden sind und jeweils ein Wasserstoffatom, ein Halogenatom oder eine Gruppe der Formel -OR⁹ bedeuten,
R³ und R⁴ gleich oder verschieden sind und jeweils eine Carbamoylgruppe oder eine Carboxygruppe bedeuten,
beide Reste R⁵ und R⁶ Wasserstoffatome darstellen,
R⁹ ein Wasserstoffatom, eine C₁-C₆-Alkylgruppe, eine Alkylsulfonylgruppe, eine Halogenalkylsulfonylgruppe, eine Arylsulfonylgruppe oder eine Hydroxy-Schutzgruppe bedeutet,
R¹² eine C₁-C₆-Alkylgruppe bedeutet,
und pharmazeutisch geeignete Salze und Ester dieser Verbindungen.

2. Verbindungen nach Anspruch 1, worin
R¹ und R² gleich oder verschieden sind und jeweils ein Wasserstoffatom, ein Halogenatom oder eine Gruppe der Formel -OR^{9a} bedeuten,
wobei R^{9a} ein Wasserstoffatom, eine Alkoxycarbonylgruppe, eine Alkenyloxycarbonylgruppe, eine Aralkyloxycarbonylgruppe, eine Acylgruppe einer aliphatischen C₁-C₂₀-Carbonsäure oder eine Acylgruppe einer carbocyclischen aromatischen Carbonsäure darstellt.

3. Verbindungen nach Anspruch 1 oder Anspruch 2, worin
R³ und R⁴ gleich oder verschieden sind und jeweils eine Carboxygruppe, eine Carbamoylgruppe, eine C₂-C₅-Alkoxycarbonylgruppe, eine (5-Methyl-2-oxo-1,3-dioxolen-4-yl)-methoxycarbonylgruppe, eine Phthalidyloxycarbonylgruppe oder eine C₂-C₅-Alkoxycarbonylgruppe, die mindestens einen unter Arylgruppen, Acyloxygruppen von C₁-C₆-aliphatischen Carbonsäuren und C₁-C₄-Alkoxycarbonyloxygruppen ausgewählten Substituenten aufweist, bedeuten.

4. Verbindungen nach Anspruch 1, worin
R¹ und R² gleich oder verschieden sind und jeweils ein Wasserstoffatom, ein Halogenatom, eine Hydroxygruppe, eine unsubstituierte aliphatische C₁-C₄-Acyloxygruppe oder eine aromatische Acyloxygruppe, deren aromatischer Teil eine C₆-C₁₀-carbocyclische Arylgruppe ist, die unsubstituiert ist oder die mindestens 1 bis 3 unter Nitrogruppen, Halogenatomen, C₁-C₄-Alkylgruppen, C₁-C₄-Alkoxygruppen, Aminogruppen, Hydroxygruppen und Cyangruppen ausgewählte Substituenten aufweist, bedeuten.

5. Verbindungen nach Anspruch 4, worin
R³ eine Carboxygruppe, eine C₂-C₅-Alkoxycarbonylgruppe, eine (5-Methyl-2-oxo-1,3-dioxolen-4-yl)-methoxycarbonylgruppe, eine Phthalidyloxycarbonylgruppe oder eine C₂-C₅-Alkoxycarbonylgruppe bedeutet, die 1 oder 2 Substituenten aufweist, die ausgewählt sind unter C₂-C₅-Alkanoyloxygruppen, C₁-C₄-Alkoxycarbonyloxygruppen und carboxyclischen C₆-C₁₀-Arylgruppen, die unsubstituiert sind oder mindestens 1 bis 3 Substituenten aufweisen, die unter Nitrogruppen, Halogenatomen, C₁-C₄-Alkylgruppen, C₁-C₄-Alkoxygruppen, Aminogruppen, Hydroxygruppen und Cyangruppen ausgewählt sind, und
R⁴ eine Carbamoylgruppe oder irgendeine der oben für R³ definierten Gruppen bedeutet.

6. Verbindungen nach Anspruch 1, worin
R¹ und R² gleich oder verschieden sind und jeweils ein Wasserstoffatom, ein Halogenatom oder eine Hydroxygruppe bedeuten.

7. Verbindungen nach Anspruch 6, worin
R³ eine Carboxygruppe, eine C₂-C₅-Alkoxycarbonylgruppe, eine (5-Methyl-2-oxo-1,3-dioxolen-4-yl)-methoxycarbonylgruppe, eine Phthalidyloxycarbonylgruppe oder eine C₂-C₅-Alkoxycarbonylgruppe bedeutet, die 1 oder 2 Substituenten aufweist, die ausgwählt sind unter C₂-C₅-Alkanoyloxygruppen, C₁-C₄-Alkoxycarbonyloxygruppen und carboxyclischen C₆-C₁₀-Arylgruppen, die unsubstituiert sind, oder 1 bis 3 Substituenten, ausgewählt unter Nitrogruppen, Halogenatomen, C₁-C₄-Alkylgruppen, C₁-C₄-Alkoxygruppen, Aminogruppen, Hydroxygruppen und Cyangruppen, aufweisen und
R⁴ eine Carbamoylgruppe oder irgendeine der oben für R³ definierten Gruppen bedeutet.

8. Arzneimittelzusammensetzung, die einen Phosphodiesteraseinhibitor im Gemisch mit einem pharmazeutisch geeigneten Trägermaterial oder Verdünnungsmittel enthält, wobei der Phosphodiesteraseinhibitor mindestens eine Verbindung gemäß einem der vorhergehenden Patentansprüche ist.

9. Verwendung einer Verbindung gemäß einem der Ansprüche 1 bis 7 zur Herstellung eines Arzneimittels zur Behandlung von Störungen, die auf ein Phosphodiesteraseungleichgewicht zurückzuführen sind.

10. Verfahren zur Herstellung von Verbindungen nach einem der Ansprüche 1 bis 7 und von pharmazeutisch geeigneten Salzen und Estern dieser Verbindungen, welches darin besteht, daß die Adenineinheit von Griseolsäure oder Dihydrodesoxygriseolsäure durch eine andere Gruppe der Formel (A) ersetzt wird und/oder, daß irgendeine der vorstehend als R¹, R², R³, R⁴, R⁵, R⁶, R⁹ und R¹² definierten Gruppen durch irgendeine andere Gruppe innerhalb der Definitionen dieser Gruppen ersetzt wird.

## Revendications (Revendications pour l'(les) Etat(s) contractant(s) suivant(s): AT)

1. Procédé de préparation de composés de formule (I) : 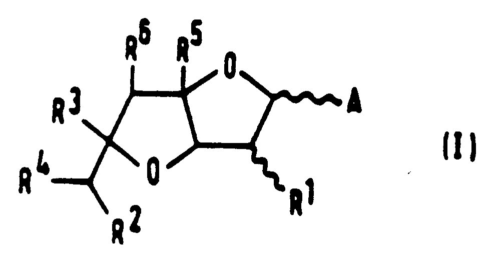 dans lequel :
A représente un groupe de formule : 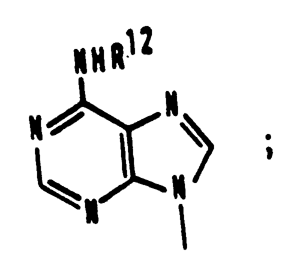 R¹ et R² sont identiques ou différents, et représentent chacun un atome d'hydrogène, un atome d'halogène ou un groupe de formule -OR⁹ ;
R³ et R⁴ sont identiques ou différents, et représentent chacun un groupe carbamoyle ou un groupe carboxy ;
R⁵ et R⁶ représentent tous les deux des atomes d'hydrogène ;
R⁹ représente un atome d'hydrogène, un groupe alkyle en C₁-C₆, un groupe alkylsulfonyle, un groupe haloalkylsulfonyle, un groupe arylsulfonyle ou un groupe hydroxy-protecteur ;
R¹² représente un groupe alkyle en C₁-C₆ ;
ainsi que les sels et les esters pharmaceutiquement acceptables dérivés de ceux-ci ;
selon lequel on remplace la partie adénine de l'acide griséolique ou de l'acide dishydrodésoxygriséolique, par un autre groupe de formule (A) et/ou, on remplace l'un des groupes R¹, R², R³, R⁴, R⁵, R⁶, R⁹ et R¹² définis ci-dessus, par un quelconque autre groupe correspondant aux définitions de ces groupes.

2. Procédé selon la revendication 1, dans lequel :
R¹ et R² sont identiques ou différents, et représentent chacun un atome d'hydrogène, un atome d'halogène ou un groupe de formule -OR^{9a},
R^{9a} représentant un atome d'hydrogène, un groupe alkoxycarbonyle, alcényloxycarbonyle, aralkyloxycarbonyle, acyle carboxylique, aliphatique en C₁-C₂₀ ou acyle carboxylique aromatique et carbocyclique.

3. Procédé selon la revendication 1 ou 2, dans lequel :
R³ et R⁴ sont identiques ou différents et représentent chacun un groupe carboxy, carbamoyle, (alkoxy en C₂-C₅) carbonyle, (5-méthyl-2-oxo-1,3-dioxolèn-4-yl)méthoxycarbonyle, phtalidyloxycarbonyle ou un groupe (alcoxy en C₂-C₅)carbonyle comportant au moins un substituant choisi parmi les groupes aryle, les groupes acyloxy carboxyliques aliphatiques en C₁-C₆ et les groupes (alkoxy en C₁-C₄) carbonyloxy.

4. Procédé selon la revendication 1, dans lequel :
R¹ et R² sont identiques ou différents, et représentent chacun un atome d'hydrogène, un atome d'halogène, un groupe hydroxy, un groupe acyloxy, aliphatique en C₁-C₄, non substitué, ou un groupe acyloxy aromatique dans lequel la partie aromatique comprend un groupe aryle carbocyclique en C₆-C₁₀ qui est non substitué, ou comporte de 1 à 3 substituants choisis parmi un groupe nitro, les atomes d'halogène, les groupes alkyle en C₁-C₄, les groupes alkoxy enC₁-C₄, un groupe amino, un groupe hydroxy et un groupe cyano.

5. Procédé selon la revendication 4, dans lequel :
R³ représente un groupe carboxy, (alkoxy en C₂-C₅)carbonyle, (5-méthyl-2-oxo-1,3-dioxolèn-4-yl)méthoxycarbonyle, phtalidyloxycarbonyle ou (alkoxy en C₂-C₅)carbonyle comportant un ou deux substituants choisis parmi les groupes alcanoyloxy en C₂-C₅ (alcoxy en C₁-C₄)carbonyloxy, et les groupes aryle carbocycliques en C₆-C₁₀ qui sont non substitués ou qui comportent de 1 à 3 substituants choisis parmi un groupe nitro, les atomes d'halogène, les groupes alkyle en C₁-C₄, les groupes alkoxy en C₁-C₄, un groupe amino, un groupe hydroxy et un groupe cyano ; et
R⁴ représente un groupe carbamoyle, ou l'un quelconque des groupes définis ci-dessus pour R³.

6. Procédé selon la revendication 1, dans lequel :
R¹ et R² sont identiques ou différents, et représentent chacun un atome d'hydrogène, un atome d'halogène ou un groupe hydroxy.

7. Procédé selon la revendication 6, dans lequel :
R³ représente un groupe carboxy, un groupe (alkoxy en C₂-C₅)carbonyle, (5-méthyl-2-oxo-1,3-dioxolèn-4-yl)-méthoxycarbonyle, phtalidyloxycarbonyle ou un groupe (alkoxy en C₂-C₅)carbonyle comportant un ou deux substituants choisis parmi les groupes alcanoyloxy en C₂-C₅ (alcoxy en C₁-C₄)carbonyloxy, et aryle carbocycliques en C₆-C₁₀ qui sont non substitués ou qui comportent de 1 à 3 substituants choisis parmi un groupe nitro, les atomes d'halogène, les groupes alkyle en C₁-C₄, les groupes alkoxy en C₁-C₄, un groupe amino, un groupe hydroxy et un groupe cyano ; et
R⁴ représente un groupe carbamoyle, ou l'un quelconque des groupes définis ci-dessus pour R³.

## Revendications (Revendications pour l'(les) Etat(s) contractant(s) suivant(s): BE, CH, LI, DE, FR, GB, IT, LU, NL, SE)

1. Composés de formule (I) : 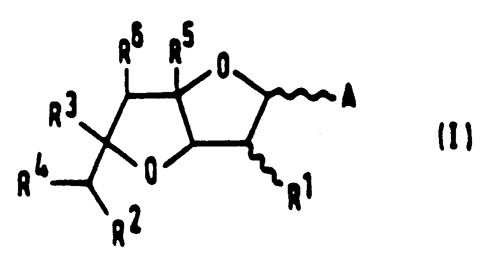 dans laquelle :
A représente un groupe de formule : 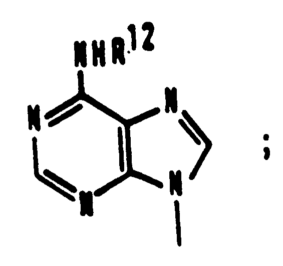 R¹ et R² sont identiques ou différents, et représentent chacun un atome d'hydrogène, un atome d'halogène ou un groupe de formule -OR⁹ ;
R³ et R⁴ sont identiques ou différents, et représentent chacun un groupe carbamoyle ou un groupe carboxy ;
R⁵ et R⁶ représentent tous les deux des atomes d'hydrogène ;
R⁹ représente un atome d'hydrogène, un groupe alkyle en C₁-C₆, un groupe alkylsulfonyle, un groupe haloalkylsulfonyle, un groupe arylsulfonyle ou un groupe hydroxy-protecteur ;
R¹² représente un groupe alkyle en C₁-C₆ ;
ainsi que les sels et les esters pharmaceutiquement acceptables dérivés de ceux-ci.

2. Composés selon la revendication 1, dans lequels :
R¹ et R² sont identiques ou différents, et représentent chacun un atome d'hydrogène, un atome d'halogène ou un groupe de formule -OR^{9a},
R^{9a} représentant un atome d'hydrogène, un groupe alkoxycarbonyle, alcényloxycarbonyle, aralkyloxycarbonyle, acyle carboxylique aliphatique en C₁-C₂₀ ou acyle carboxylique aromatique carbocyclique.

3. Composés selon la revendication 1 ou 2, dans lesquels :
R³ et R⁴ sont identiques ou différents et représentent chacun un groupe carboxy, carbamoyle, (alkoxy en C₂-C₅) carbonyle, (5-méthyl-2-oxo-1,3-dioxolèn-4-yl)méthoxycarbonyle, phtalidyloxycarbonyle ou un groupe (alcoxy en C₂-C₅)carbonyle comportant au moins un substituant choisi parmi les groupes aryle, les groupes acyloxy carboxyliques aliphatiques en C₁-C₆ et les groupes (alkoxy en C₁-C₄) carbonyloxy.

4. Composés selon la revendication 1, dans lesquels :
R¹ et R² sont identiques ou différents, et représentent chacun un atome d'hydrogène, un atome d'halogène, un groupe hydroxy, un groupe acyloxy, aliphatique en C₁-C₄, non substitué, ou un groupe acyloxy aromatique dans lequel la partie aromatique comprend un groupe aryle carbocyclique en C₆-C₁₀ qui est non substitué, ou comporte de 1 à 3 substituants choisis parmi un groupe nitro, les atomes d'halogène, les groupes alkyle en C₁-C₄, les groupes alkoxy en C₁-C₄, un groupe amino, un groupe hydroxy et un groupe cyano.

5. Composés selon la revendication 4, dans lesquels :
R³ représente un groupe carboxy, (alkoxy en C₂-C₅)carbonyle, (5-méthyl-2-oxo-1,3-dioxolèn-4-yl)méthoxycarbonyle, phtalidyloxycarbonyle ou (alkoxy en C₂-C₅)carbonyle comportant un ou deux substituants choisis parmi les groupes alcanoyloxy en C₂-C₅ (alcoxy en C₁-C₄)carbonyloxy, et les groupes aryle carbocycliques en C₆-C₁₀ qui sont non substitués ou qui comportent de 1 à 3 substituants choisis parmi un groupe nitro, les atomes d'halogène, les groupes alkyle en C₁-C₄, les groupes alkoxy en C₁-C₄, un groupe amino, un groupe hydroxy et un groupe cyano ; et
R⁴ représente un groupe carbamoyle, ou l'un quelconque des groupes définis ci-dessus pour R³.

6. Composés selon la revendication 1, dans lesquels :
R¹ et R² sont identiques ou différents, et représentent chacun un atome d'hydrogène, un atome d'halogène ou un groupe hydroxy.

7. Composés selon la revendication 6, dans lesquels :
R³ représente un groupe carboxy, un groupe (alkoxy en C₂-C₅)carbonyle, (5-méthyl-2-oxo-1,3-dioxolèn-4-yl)-méthoxycarbonyle, phtalidyloxycarbonyle ou un groupe (alkoxy en C₂-C₅)carbonyle comportant un ou deux substituants choisis parmi les groupes alcanoyloxy en C₂-C₅ (alcoxy en C₁-C₄)carbonyloxy, et aryle carbocycliques en C₆-C₁₀ qui sont non substitués ou qui comportent de 1 à 3 substituants choisis parmi un groupe nitro, les atomes d'halogène, les groupes alkyle en C₁-C₄, les groupes alkoxy en C₁-C₄, un groupe amino, un groupe hydroxy et un groupe cyano ; et
R⁴ représente un groupe carbamoyle, ou l'un quelconque des groupes définis ci-dessus pour R³.

8. Composition pharmaceutique, comprenant un inhibiteur de phosphodiestérase en mélange avec un véhicule ou un diluant pharmaceutiquement acceptable, l'inhibiteur de phosphodiestérase comprenant au moins un composé selon l'une quelconque des revendications précédentes.

9. Utilisation, pour la fabrication d'un médicament destiné au traitement de troubles dus à un déséquilibre de la phosphodiétérase, d'un composé selon l'une quelconque des revendications 1 à 7.

10. Procédé de préparation de composés selon l'une quelconque des revendications 1 à 7, et de sels ainsi que d'esters pharmaceutiquement acceptables dérivés de ceux-ci, selon lequel on remplace la partie adénine de l'acide griséolique ou de l'acide dihydrodésoxygriséolique, par un autre groupe de formule (A) et/ou, on remplace l'un des groupes R¹, R², R³, R⁴, R⁵, R⁶, R⁹ et R¹² définis ci-dessus, par un quelconque autre groupe correspondant aux définitions de ces groupes.
